# FASCICULE DE BREVET EUROPEEN

(11) **EP 2 029 748 B1**
(45) Date de publication et mention de la délivrance du brevet: **16.11.2016**
(21) Numéro de dépôt: 07731315.3
(22) Date de dépôt: 18.04.2007
(51) Int. Cl.: C12N 15/62, C12N 15/45, C07K 14/135, A61K 39/155, C07K 14/115, A61K 47/48, A61K 39/00, C07K 14/005

(54) **PROTEINES DE FUSION PROTEINE N D'UN VIRUS DE LA FAMILLE DES PARAMYXOVIRIDAE-PROTEINE D'INTERET**
FUSIONSPROTEINE ZWISCHEN EINEN N-PROTEIN EINES VIRUS AUS DER FAMILIE DER PARAMYOVIRIDAE UND EINEN INTERESTPROTEIN
N PROTEIN FUSION PROTEINS OF A VIRUS IN THE PARAMYXOVIRIDAE-PROTEIN OF INTEREST FAMILY AND THEIR USES FOR VACCINATION AND INTRA-CELLULAR VECTORISATION

(30) Priorité: 18.04.2006 FR 0603410
(43) Date de publication de la demande: 04.03.2009
(73) Titulaire: Institut National de la Recherche Agronomique, 75338 Paris Cedex 07 (FR)
(72) Inventeur: ELEOUËT, Jean-François, 91650 Breuillet (FR); RIFFAULT, Sabine, 78350 Jouy En Josas (FR)
(74) Mandataire: Lavoix
(86) Numéro de dépôt international: PCT/FR2007/000651
(87) Numéro de publication internationale: WO 2007/119011

(56) Documents cités:
- WO-A-95/27787
- WO-A-2006/117456
- WO-A2-2004/087062
- CASTAGNE N ET AL: "Biochemical characterization of the respiratory syncytial virus P-P and P-N protein complexes and localization of the P protein oligomerization domain" JOURNAL OF GENERAL VIROLOGY, SOCIETY FOR GENERAL MICROBIOLOGY, SPENCERS WOOD, GB, vol. 85, no. Part 6, juin 2004 (2004-06), pages 1643-1653, XP002361372 ISSN: 0022-1317
- KHATTAR S K ET AL: "Mutational Analysis of the Bovine Respiratory Syncytial Virus Nucleocapsid Protein Using a Minigenome System: Mutations That Affect Encapsidation, RNA Synthesis, and Interaction with the Phosphoprotein" VIROLOGY, ACADEMIC PRESS,ORLANDO, US, vol. 270, no. 1, 25 avril 2000 (2000-04-25), pages 215-228, XP004436061 ISSN: 0042-6822
- MURPHY LB, LONEY C, MURRAY J, BHELLA D, ASHTON P, YEO RP.: "nvestigations into the amino-terminal domain of the respiratory syncytial virus nucleocapsid protein reveal elements important for nucleocapsid formation and interaction with the phosphoprotein" VIROLOGY., vol. 307, no. 1, 1 mars 2003 (2003-03-01), XP002449466
- MAVRAKIS ET AL: "Rabies virus chaperone: Identification of the phosphoprotein peptide that keeps nucleoprotein soluble and free from non-specific RNA" VIROLOGY, ACADEMIC PRESS,ORLANDO, US, vol. 349, no. 2, 21 février 2006 (2006-02-21), - 5 juin 2006 (2006-06-05) pages 422-429, XP005469133 ISSN: 0042-6822
- KHATTAR S K ET AL: "Mapping the domains on the phosphoprotein of bovine respiratory syncytial virus required for N-P and P-L interactions using a minigenome system" JOURNAL OF GENERAL VIROLOGY, SOCIETY FOR GENERAL MICROBIOLOGY, SPENCERS WOOD, GB, vol. 82, no. 4, avril 2001 (2001-04), pages 775-779, XP002361379 ISSN: 0022-1317
- CHOI KS, NAH JJ, KO YJ, KANG SY,: "Rapid competitive enzyme-linked immunosorbent assay for detection of antibodies to peste des petits ruminants virus." JO NI.CLIN DIAGN LAB IMMUNOL., vol. 12, no. 4, avril 2005 (2005-04), pages 542-547, XP002462213
- BECK A, ZORN N, BUSSAT MC, HAEUW JF, CORVAÏA N, NGUYEN TN, BONNEFOY JY, VAN DORSSELAER A.: "Synthesis and characterization of Respiratory Syncytial Virus protein G related peptides containing two disulfide bridges" J PEPT RES., vol. 55, no. 1, janvier 2000 (2000-01), pages 24-35, XP002462214
- CHEN I ET AL: "Site-specific labeling of proteins with small molecules in live cells" CURRENT OPINION IN BIOTECHNOLOGY, LONDON, GB, vol. 16, no. 1, février 2005 (2005-02), pages 35-40, XP004756194 ISSN: 0958-1669
- STITTELAAR KJ, HOOGERHOUT P, OVAA W, VAN BINNENDIJK RR, POELEN MC, ROHOLL P, VAN ELS CA, OSTERHAUS AD, WIERTZ EJ: "In vitro processing and presentation of a lipidated cytotoxic T-cell epitope derived from measles virus fusion protein." VACCINE., vol. 20, no. 1-2, 12 octobre 2001 (2001-10-12), pages 249-261, XP002462215
- THI-LAN TRAN ET AL: "The nine C-terminal amino acids of the respiratory syncytial virus protein P are necessary and sufficient for binding to ribonucleoprotein complexes in which six ribonucleotides are contacted per N protein protomer", JOURNAL OF GENERAL VIROLOGY, SOCIETY FOR GENERAL MICROBIOLOGY, SPENCERS WOOD, GB, vol. 88, no. Part 1, 1 January 2007 (2007-01-01), pages 196-206, XP002622450, ISSN: 0022-1317, DOI: 10.1099/VIR.0.82282-0
- SIVASAMUGHAM LALITA AMBIGAI ET AL: "Recombinant Newcastle disease virus capsids displaying enterovirus 71 VP1 fragment induce a strong immune response in rabbits", JOURNAL OF MEDICAL VIROLOGY, JOHN WILEY & SONS, INC, US, vol. 78, no. 8, 20 June 2006 (2006-06-20), pages 1096-1104, XP002403705, ISSN: 0146-6615, DOI: 10.1002/JMV.20668
- RIMANTAS SLIBINSKAS ET AL: "Synthesis of the measles virus nucleoprotein in yeast Pichia pastoris and Saccharomyces cerevisiae", JOURNAL OF BIOTECHNOLOGY, vol. 107, no. 2, 1 January 2004 (2004-01-01), pages 115-124, XP055102902, ISSN: 0168-1656, DOI: 10.1016/j.jbiotec.2003.10.018
- KRISTER BLODÖRN ET AL: 'Vaccine Safety and Efficacy Evaluation of a Recombinant Bovine Respiratory Syncytial Virus (BRSV) with Deletion of the SH Gene and Subunit Vaccines Based On Recombinant Human RSV Proteins: N-nanorings, P and M2-1, in Calves with Maternal Antibodies' PLOS ONE vol. 9, no. 6, E100392, 19 Juin 2014, pages 1 - 17, XP055171192 DOI: 10.1371/journal.pone.0100392
- XAVIER ROUX ET AL: 'Sub-Nucleocapsid Nanoparticles: A Nasal Vaccine against Respiratory Syncytial Virus' PLOS ONE vol. 3, no. 3, E1766, 01 Janvier 2008, pages 1 - 14, XP055008640 DOI: 10.1371/journal.pone.0001766 ISSN: 1932-6203
- P.-L. Herve ET AL: "A Novel Subnucleocapsid Nanoplatform for Mucosal Vaccination against Influenza Virus That Targets the Ectodomain of Matrix Protein 2", Journal of Virology, vol. 88, no. 1, 23 October 2013 (2013-10-23), pages 325-338, XP055169930, ISSN: 0022-538X, DOI: 10.1128/JVI.01141-13
- Sabine Riffault ET AL: "A new subunit vaccine based on nucleoprotein nanoparticles confers partial clinical and virological protection in calves against bovine respiratory syncytial virus", Vaccine, vol. 28, no. 21, 1 May 2010 (2010-05-01), pages 3722-3734, XP055169935, ISSN: 0264-410X, DOI: 10.1016/j.vaccine.2010.03.008
- ROCHE: 'RTS Application Manual ofr cell-free protein expression', Bd. '2.2. Tags used for expression of proteins', pages 40 - 42

### SEQUENCE LISTING

<110> Institut National de la Recherche Agronomique
<120> Protéines de fusion protéine N d'un virus de la famille des Paramyxoviridae - protéine d'intérêt et leurs utilisations pour la vaccination et la vectorisation intra-cellulaire
<130> BET07P0251
<150> FR0603410
   <151> 2006-04-18
<160> 35
<170> PatentIn version 3.3
<210> 1
   <211> 391
   <212> PRT
   <213> Human respiratory syncytial virus
<400> 1
   Met Ala Leu Ser Lys Val Lys Leu Asn Asp Thr Leu Asn Lys Asp Gln
   1               5                   10                  15
   Leu Leu Ser Ser Ser Lys Tyr Thr Ile Gln Arg Ser Thr Gly Asp Ser
               20                  25                  30
   Ile Asp Thr Pro Asn Tyr Asp Val Gln Lys His Ile Asn Lys Leu Cys
           35                  40                  45
   Gly Met Leu Leu Ile Thr Glu Asp Ala Asn His Lys Phe Thr Gly Leu
       50                  55                  60
   Ile Gly Met Leu Tyr Ala Met Ser Arg Leu Gly Arg Glu Asp Thr Ile
   65                  70                  75                  80
   Lys Ile Leu Arg Asp Ala Gly Tyr His Val Lys Ala Asn Gly Val Asp
                   85                  90                  95
   Val Thr Thr His Arg Gln Asp Ile Asn Gly Lys Glu Met Lys Phe Glu
               100                 105                 110
   Val Leu Thr Leu Ser Ser Leu Thr Thr Glu Ile Gln Ile Asn Ile Glu
           115                 120                 125
   Ile Glu Ser Arg Lys Ser Tyr Lys Lys Met Leu Lys Glu Met Gly Glu
       130                 135                 140
   Val Ala Pro Glu Tyr Arg His Asp Ser Pro Asp Cys Gly Met Ile Ile
   145                 150                 155                 160
   Leu Cys Ile Ala Ala Leu Val Ile Thr Lys Leu Ala Ala Gly Asp Arg
                   165                 170                 175
   Ser Gly Leu Thr Ala Val Ile Arg Arg Ala Asn Asn Val Leu Lys Asn
               180                 185                 190
   Glu Met Lys Arg Tyr Lys Gly Leu Leu Pro Lys Asp Ile Ala Asn Ser
           195                 200                 205
   Phe Tyr Glu Val Phe Glu Lys Tyr Pro His Phe Ile Asp Val Phe Val
       210                 215                 220
   His Phe Gly Ile Ala Gln Ser Ser Thr Arg Gly Gly Ser Arg Val Glu
   225                 230                 235                 240
   Gly Ile Phe Ala Gly Leu Phe Met Asn Ala Tyr Gly Ala Gly Gln Val
                   245                 250                 255
   Met Leu Arg Trp Gly Val Leu Ala Lys Ser Val Lys Asn Ile Met Leu
               260                 265                 270
   Gly His Ala Ser Val Gln Ala Glu Met Glu Gln Val Val Glu Val Tyr
           275                 280                 285
   Glu Tyr Ala Gln Lys Leu Gly Gly Glu Ala Gly Phe Tyr His Ile Leu
       290                 295                 300
   Asn Asn Pro Lys Ala Ser Leu Leu Ser Leu Thr Gln Phe Pro His Phe
   305                 310                 315                 320
   Ser Ser Val Val Leu Gly Asn Ala Ala Gly Leu Gly Ile Met Gly Glu
                   325                 330                 335
   Tyr Arg Gly Thr Pro Arg Asn Gln Asp Leu Tyr Asp Ala Ala Lys Ala
               340                 345                 350
   Tyr Ala Glu Gln Leu Lys Glu Asn Gly Val Ile Asn Tyr Ser Val Leu
           355                 360                 365
   Asp Leu Thr Ala Glu Glu Leu Glu Ala Ile Lys His Gln Leu Asn Pro
       370                 375                 380
   Lys Asp Asn Asp Val Glu Leu
   385                 390
<210> 2
   <211> 391
   <212> PRT
   <213> Bovine respiratory syncytial virus
<400> 2
   Met Ala Leu Ser Lys Val Lys Leu Asn Asp Thr Phe Asn Lys Asp Gln
   1               5                   10                  15
   Leu Leu Ser Thr Ser Lys Tyr Thr Ile Gln Arg Ser Thr Gly Asp Asn
               20                  25                  30
   Ile Asp Ile Pro Asn Tyr Asp Val Gln Lys His Leu Asn Lys Leu Cys
           35                  40                  45
   Gly Met Leu Leu Ile Thr Glu Asp Ala Asn His Lys Phe Thr Gly Leu
       50                  55                  60
   Ile Gly Met Leu Tyr Ala Met Ser Arg Leu Gly Arg Glu Asp Thr Leu
   65                  70                  75                  80
   Lys Ile Leu Lys Asp Ala Gly Tyr Gln Val Arg Ala Asn Gly Val Asp
                   85                  90                  95
   Val Ile Thr His Arg Gln Asp Val Asn Gly Lys Glu Met Lys Phe Glu
               100                 105                 110
   Val Leu Thr Leu Val Ser Leu Thr Ser Glu Val Gln Gly Asn Ile Glu
           115                 120                 125
   Ile Glu Ser Arg Lys Ser Tyr Lys Lys Met Leu Lys Glu Met Gly Glu
       130                 135                 140
   Val Ala Pro Glu Tyr Arg His Asp Phe Pro Asp Cys Gly Met Ile Val
   145                 150                 155                 160
   Leu Cys Val Ala Ala Leu Val Ile Thr Lys Leu Ala Ala Gly Asp Arg
                   165                 170                 175
   Ser Gly Leu Thr Ala Val Ile Arg Arg Ala Asn Asn Val Leu Arg Asn
               180                 185                 190
   Glu Met Lys Arg Tyr Lys Gly Leu Ile Pro Lys Asp Ile Ala Asn Ser
           195                 200                 205
   Phe Tyr Glu Val Phe Glu Lys Tyr Pro His Tyr Ile Asp Val Phe Val
       210                 215                 220
   His Phe Gly Ile Ala Gln Ser Ser Thr Arg Gly Gly Ser Arg Val Glu
   225                 230                 235                 240
   Gly Ile Phe Ala Gly Leu Phe Met Asn Ala Tyr Gly Ala Gly Gln Val
                   245                 250                 255
   Met Leu Arg Trp Gly Val Leu Ala Lys Ser Val Lys Asn Ile Met Leu
               260                 265                 270
   Gly His Ala Ser Val Gln Ala Glu Met Glu Gln Val Val Glu Val Tyr
           275                 280                 285
   Glu Tyr Ala Gln Lys Leu Gly Gly Glu Ala Gly Phe Tyr His Ile Leu
       290                 295                 300
   Asn Asn Pro Lys Ala Ser Leu Leu Ser Leu Thr Gln Phe Pro Asn Phe
   305                 310                 315                 320
   Ser Ser Val Val Leu Gly Asn Ala Ala Gly Leu Gly Ile Met Gly Glu
                   325                 330                 335
   Tyr Arg Gly Thr Pro Arg Asn Gln Asp Leu Tyr Asp Ala Ala Lys Ala
               340                 345                 350
   Tyr Ala Glu Gln Leu Lys Glu Asn Gly Val Ile Asn Tyr Ser Val Leu
           355                 360                 365
   Asp Leu Thr Thr Glu Glu Leu Glu Ala Ile Lys Asn Gln Leu Asn Pro
       370                 375                 380
   Lys Asp Asn Asp Val Glu Leu
   385                 390
<210> 3
   <211> 524
   <212> PRT
   <213> Sendai virus
<400> 3
   Met Ala Gly Leu Leu Ser Thr Phe Asp Thr Phe Ser Ser Arg Arg Ser
   1               5                   10                  15
   Glu Ser Ile Asn Lys Ser Gly Gly Gly Ala Val Ile Pro Gly Gln Arg
               20                  25                  30
   Ser Thr Val Ser Val Phe Val Leu Gly Pro Ser Val Thr Asp Asp Ala
   35 40 45
   Asp Lys Leu Phe Ile Ala Thr Thr Phe Leu Ala His Ser Leu Asp Thr
   50 55 60
   Asp Lys Gln His Ser Gln Arg Gly Gly Phe Leu Val Ser Leu Leu Ala
   65 70 75 80
   Met Ala Tyr Ser Ser Pro Glu Leu Tyr Leu Thr Thr Asn Gly Val Asn
   85 90 95
   Ala Asp Val Lys Tyr Val Ile Tyr Asn Ile Glu Lys Asp Pro Lys Arg
   100 105 110
   Thr Lys Thr Asp Gly Phe Ile Val Lys Thr Arg Asp Met Glu Tyr Glu
   115 120 125
   Arg Thr Thr Glu Trp Leu Phe Gly Pro Met Val Asn Lys Ser Pro Leu
   130 135 140
   Phe Gln Gly Gln Arg Asp Ala Ala Asp Pro Asp Thr Leu Leu Gln Ile
   145 150 155 160
   Tyr Gly Tyr Pro Ala Cys Leu Gly Ala Ile Ile Val Gln Val Trp Ile
   165 170 175
   Val Leu Val Lys Ala Ile Thr Ser Ser Ala Gly Leu Arg Lys Gly Phe
   180 185 190
   Phe Asn Arg Leu Glu Ala Phe Arg Gln Asp Gly Thr Val Lys Gly Ala
   195 200 205
   Leu Val Phe Thr Gly Glu Thr Val Glu Gly Ile Gly Ser Val Met Arg
   210 215 220
   Ser Gln Gln Ser Leu Val Ser Leu Met Val Glu Thr Leu Val Thr Met
   225 230 235 240
   Asn Thr Ala Arg Ser Asp Leu Thr Thr Leu Glu Lys Asn Ile Gln Ile
   245 250 255
   Val Gly Asn Tyr Ile Arg Asp Ala Gly Leu Ala Ser Phe Met Asn Thr
   260 265 270
   Ile Lys Tyr Gly Val Glu Thr Lys Met Ala Ala Leu Thr Leu Ser Asn
           275                 280                 285
   Leu Arg Pro Asp Ile Asn Lys Leu Arg Ser Leu Ile Asp Thr Tyr Leu
       290                 295                 300
   Ser Lys Gly Pro Arg Ala Pro Phe Ile Cys Ile Leu Lys Asp Pro Val
   305                 310                 315                 320
   His Gly Glu Phe Ala Pro Gly Asn Tyr Pro Ala Leu Trp Ser Tyr Ala
                   325                 330                 335
   Met Gly Val Ala Val Val Gln Asn Lys Ala Met Gln Gln Tyr Val Thr
               340                 345                 350
   Gly Arg Thr Tyr Leu Asp Met Glu Met Phe Leu Leu Gly Gln Ala Val
           355                 360                 365
   Ala Lys Asp Ala Glu Ser Lys Ile Ser Ser Ala Leu Glu Asp Glu Leu
       370                 375                 380
   Gly Val Thr Asp Thr Ala Lys Glu Arg Leu Arg His His Leu Ala Asn
   385                 390                 395                 400
   Leu Ser Gly Gly Asp Gly Ala Tyr His Lys Pro Thr Gly Gly Gly Ala
                   405                 410                 415
   Ile Glu Val Ala Leu Asp Asn Ala Asp Ile Asp Leu Glu Pro Glu Ala
               420                 425                 430
   His Thr Asp Gln Asp Ala Arg Gly Trp Gly Gly Asp Ser Gly Asp Arg
           435                 440                 445
   Trp Ala Arg Ser Thr Ser Ser Gly His Phe Ile Thr Leu His Gly Ala
       450                 455                 460
   Glu Arg Leu Glu Glu Glu Thr Asn Asp Glu Asp Val Ser Asp Ile Glu
   465                 470                 475                 480
   Arg Arg Ile Ala Arg Arg Leu Ala Glu Arg Arg Gln Glu Asp Ala Thr
                   485                 490                 495
   Thr His Glu Asp Glu Gly Arg Asn Asn Gly Val Asp His Asp Glu Glu
               500                 505                 510
   Asp Asp Ala Ala Ala Ala Ala Gly Met Gly Gly Ile
   515 520
<210> 4
   <211> 525
   <212> PRT
   <213> Measles virus
<400> 4
   Met Ala Thr Leu Leu Arg Ser Leu Ala Leu Phe Lys Arg Asn Lys Asp
   1               5                   10                  15
   Lys Pro Pro Ile Thr Ser Gly Ser Gly Gly Ala Ile Arg Gly Ile Lys
               20                  25                  30
   His Ile Ile Ile Val Pro Ile Pro Gly Asp Ser Ser Ile Thr Thr Arg
           35                  40                  45
   Ser Arg Leu Leu Asp Arg Leu Val Arg Leu Ile Gly Asn Pro Asp Val
       50                  55                  60
   Ser Gly Pro Lys Leu Thr Gly Ala Leu Ile Gly Ile Leu Ser Leu Phe
   65                  70                  75                  80
   Val Glu Ser Pro Gly Gln Leu Ile Gln Arg Ile Thr Asp Asp Pro Asp
                   85                  90                  95
   Val Ser Ile Arg Leu Leu Glu Val Val Gln Ser Asp Gln Ser Gln Ser
               100                 105                 110
   Gly Leu Thr Phe Ala Ser Arg Gly Thr Asn Met Glu Asp Glu Ala Asp
           115                 120                 125
   Gln Tyr Phe Ser His Asp Asp Pro Ile Ser Ser Asp Gln Ser Arg Phe
       130                 135                 140
   Gly Trp Phe Glu Asn Lys Glu Ile Ser Asp Ile Glu Val Gln Asp Pro
   145                 150                 155                 160
   Glu Gly Phe Asn Met Ile Leu Gly Thr Ile Leu Ala Gln Ile Trp Val
                   165                 170                 175
   Leu Leu Ala Lys Ala Val Thr Ala Pro Asp Thr Ala Ala Asp Ser Glu
               180                 185                 190
   Leu Arg Arg Trp Ile Lys Tyr Thr Gln Gln Arg Arg Val Val Gly Glu
           195                 200                 205
   Phe Arg Leu Glu Arg Lys Trp Leu Asp Val Val Arg Asn Arg Ile Ala
       210                 215                 220
   Glu Asp Leu Ser Leu Arg Arg Phe Met Val Ala Leu Ile Leu Asp Ile
   225                 230                 235                 240
   Lys Arg Thr Pro Gly Asn Lys Pro Arg Ile Ala Glu Met Ile Cys Asp
                   245                 250                 255
   Ile Asp Thr Tyr Ile Val Glu Ala Gly Leu Ala Ser Phe Ile Leu Thr
               260                 265                 270
   Ile Lys Phe Gly Ile Glu Thr Met Tyr Pro Ala Leu Gly Leu His Glu
           275                 280                 285
   Phe Ala Gly Glu Leu Ser Thr Leu Glu Ser Leu Met Asn Leu Tyr Gln
       290                 295                 300
   Gln Met Gly Glu Thr Ala Pro Tyr Met Val Ile Leu Glu Asn Ser Ile
   305                 310                 315                 320
   Gln Asn Lys Phe Ser Ala Gly Ser Tyr Pro Leu Leu Trp Ser Tyr Ala
                   325                 330                 335
   Met Gly Val Gly Val Glu Leu Glu Asn Ser Met Gly Gly Leu Asn Phe
               340                 345                 350
   Gly Arg Ser Tyr Phe Asp Pro Ala Tyr Phe Arg Leu Gly Gln Glu Met
           355                 360                 365
   Val Arg Arg Ser Ala Gly Lys Val Ser Ser Thr Leu Ala Ser Glu Leu
       370                 375                 380
   Gly Ile Thr Ala Glu Asp Ala Arg Leu Val Ser Glu Ile Ala Met His
   385                 390                 395                 400
   Thr Thr Glu Asp Lys Ile Ser Arg Ala Val Gly Pro Arg Gln Ala Gln
                   405                 410                 415
   Val Ser Phe Leu His Gly Asp Gln Ser Glu Asn Glu Leu Pro Arg Leu
               420                 425                 430
   Gly Gly Lys Glu Asp Arg Arg Val Lys Gln Ser Arg Gly Glu Ala Arg
           435                 440                 445
   Glu Ser Tyr Arg Glu Thr Gly Pro Ser Arg Ala Ser Asp Ala Arg Ala
   450 455 460
   Ala His Leu Pro Thr Gly Thr Pro Leu Asp Ile Asp Thr Ala Ser Glu
   465 470 475 480
   Ser Ser Gln Asp Pro Gln Asp Ser Arg Arg Ser Ala Asp Ala Leu Leu
   485 490 495
   Arg Leu Gln Ala Met Ala Gly Ile Ser Glu Glu Gln Gly Ser Asp Thr
   500 505 510
   Asp Thr Pro Ile Val Tyr Asn Asp Arg Asn Leu Leu Asp
   515 520 525
<210> 5
   <211> 553
   <212> PRT
   <213> Mumps virus
<400> 5
   Met Ser Ser Val Leu Lys Ala Phe Glu Arg Phe Thr Ile Glu Gln Glu
   1 5 10 15
   Leu Gln Asp Arg Gly Glu Glu Gly Ser Ile Pro Pro Glu Thr Leu Lys
   20 25 30
   Ser Ala Val Lys Val Phe Val Ile Asn Thr Pro Asn Pro Thr Thr Arg
   35 40 45
   Tyr Gln Met Leu Asn Phe Cys Leu Arg Ile Ile Cys Ser Gln Asn Arg
   50 55 60
   Arg Ala Ser His Arg Val Gly Ala Leu Ile Ala Leu Phe Ser Leu Pro
   65 70 75 80
   Ser Ala Gly Met Gln Asn His Ile Arg Leu Ala Asp Arg Ser Pro Glu
   85 90 95
   Ala Gln Ile Glu Arg Cys Glu Ile Asp Gly Phe Glu Pro Gly Thr Tyr
   100 105 110
   Arg Leu Ile Pro Asn Ala Arg Ala Asn Leu Thr Ala Asn Glu Ile Ala
   115 120 125
   Ala Tyr Ala Leu Leu Ala Asp Asp Leu Pro Pro Thr Ile Asn Asn Gly
   130 135 140
   Thr Pro Tyr Val His Ala Asp Val Glu Leu Gln Pro Cys Asp Glu Ile
   145 150 155 160
   Glu Gln Phe Leu Asp Arg Cys Tyr Ser Val Leu Ile Gln Ala Trp Val
   165 170 175
   Met Val Cys Lys Cys Met Thr Ala Tyr Asp Gln Pro Ala Gly Ser Ala
   180 185 190
   Asp Arg Arg Phe Ala Lys Tyr Gln Gln Gln Gly Arg Leu Glu Ala Arg
   195 200 205
   Tyr Met Leu Gln Pro Glu Ala Gln Arg Leu Ile Gln Thr Ala Ile Arg
   210 215 220
   Lys Ser Leu Val Val Arg Gln Tyr Leu Thr Phe Glu Leu Gln Leu Ala
   225 230 235 240
   Arg Arg Gln Gly Leu Leu Ser Asn Arg Tyr Tyr Ala Met Val Gly Asp
   245 250 255
   Ile Gly Lys Tyr Ile Glu Asn Ser Gly Leu Thr Ala Phe Phe Leu Thr
   260 265 270
   Leu Lys Tyr Ala Leu Gly Thr Lys Trp Ser Pro Leu Ser Leu Ala Ala
   275 280 285
   Phe Thr Gly Glu Leu Thr Lys Leu Arg Ser Leu Met Met Leu Tyr Arg
   290 295 300
   Asp Ile Gly Glu Gln Ala Arg Tyr Leu Ala Leu Leu Glu Ala Pro Gln
   305 310 315 320
   Ile Met Asp Phe Ala Pro Gly Gly Tyr Pro Leu Ile Phe Ser Tyr Ala
   325 330 335
   Met Gly Val Gly Ser Val Leu Asp Val Gln Met Arg Asn Tyr Thr Tyr
   340 345 350
   Ala Arg Pro Phe Leu Asn Gly Tyr Tyr Phe Gln Ile Gly Val Glu Thr
   355 360 365
   Ala Arg Arg Gln Gln Gly Thr Val Asp Asn Arg Val Ala Asp Asp Leu
   370 375 380
   Gly Leu Thr Pro Glu Gln Arg Asn Glu Val Thr Gln Leu Val Asp Arg
   385                 390                 395                 400
   Leu Ala Arg Gly Arg Gly Ala Gly Ile Pro Gly Gly Pro Val Asn Pro
                   405                 410                 415
   Phe Val Pro Pro Val Gln Gln Gln Gln Pro Ala Ala Val Tyr Ala Asp
               420                 425                 430
   Ile Pro Ala Leu Glu Glu Ser Asp Asp Asp Gly Asp Glu Asp Gly Gly
           435                 440                 445
   Ala Gly Phe Gln Asn Gly Val Gln Val Pro Ala Val Arg Gln Gly Gly
       450                 455                 460
   Gln Thr Asp Phe Arg Ala Gln Pro Leu Gln Asp Pro Ile Gln Ala Gln
   465                 470                 475                 480
   Leu Phe Met Pro Leu Tyr Pro Gln Val Ser Asn Ile Pro Asn Asn Arg
                   485                 490                 495
   Ile Ile Arg Ser Ile Ala Ser Gly Gly Trp Lys Thr Lys Ile Tyr Tyr
               500                 505                 510
   Asp Thr Thr Arg Met Val Ile Leu Asn Lys Met Gln Gly Ala Asn Thr
           515                 520                 525
   Glu Thr Leu Ser Gln Thr Ile Pro Ile Lys Thr His Ser Cys Lys Trp
       530                 535                 540
   Ala Thr Gly Met Ser Lys Ser Leu Thr
   545                 550
<210> 6
   <211> 394
   <212> PRT
   <213> Human Metapneumovirus
<400> 6
   Met Ser Leu Gln Gly Ile His Leu Ser Asp Leu Ser Tyr Lys His Ala
   1               5                   10                  15
   Ile Leu Lys Glu Ser Gln Tyr Thr Ile Lys Arg Asp Val Gly Thr Thr
               20                  25                  30
   Thr Ala Val Thr Pro Ser Ser Leu Gln Gln Glu Ile Thr Leu Leu Cys
   35 40 45
   Gly Glu Ile Leu Tyr Ala Lys His Ala Asp Tyr Lys Tyr Ala Ala Glu
   50 55 60
   Ile Gly Ile Gln Tyr Ile Ser Thr Ala Leu Gly Ser Glu Arg Val Gln
   65 70 75 80
   Gln Ile Leu Arg Asn Ser Gly Ser Glu Val Gln Val Val Leu Thr Arg
   85 90 95
   Thr Tyr Ser Leu Gly Lys Ile Lys Asn Asn Lys Gly Glu Asp Leu Gln
   100 105 110
   Met Leu Asp Ile His Gly Val Glu Lys Ser Trp Val Glu Glu Ile Asp
   115 120 125
   Lys Glu Ala Arg Lys Thr Met Ala Thr Leu Leu Lys Glu Ser Ser Gly
   130 135 140
   Asn Ile Pro Gln Asn Gln Arg Pro Ser Ala Pro Asp Thr Pro Ile Ile
   145 150 155 160
   Leu Leu Cys Val Gly Ala Leu Ile Phe Thr Lys Leu Ala Ser Thr Ile
   165 170 175
   Glu Val Gly Leu Glu Thr Thr Val Arg Arg Ala Asn Arg Val Leu Ser
   180 185 190
   Asp Ala Leu Lys Arg Tyr Pro Arg Met Asp Ile Pro Lys Ile Ala Arg
   195 200 205
   Ser Phe Tyr Asp Leu Phe Glu Gln Lys Val Tyr His Arg Ser Leu Phe
   210 215 220
   Ile Glu Tyr Gly Lys Ala Leu Gly Ser Ser Ser Thr Gly Ser Lys Ala
   225 230 235 240
   Glu Ser Leu Phe Val Asn Ile Phe Met Gln Ala Tyr Gly Ala Gly Gln
   245 250 255
   Thr Met Leu Arg Trp Gly Val Ile Ala Arg Ser Ser Asn Asn Ile Met
   260 265 270
   Leu Gly His Val Ser Val Gln Ala Glu Leu Lys Gln Val Thr Glu Val
   275 280 285
   Tyr Asp Leu Val Arg Glu Met Gly Pro Glu Ser Gly Leu Leu His Leu
   290 295 300
   Arg Gln Ser Pro Lys Ala Gly Leu Leu Ser Leu Ala Asn Cys Pro Asn
   305 310 315 320
   Phe Ala Ser Val Val Leu Gly Asn Ala Ser Gly Leu Gly Ile Ile Gly
   325 330 335
   Met Tyr Arg Gly Arg Val Pro Asn Thr Glu Leu Phe Ser Ala Ala Glu
   340 345 350
   Ser Tyr Ala Lys Ser Leu Lys Glu Ser Asn Lys Ile Asn Phe Ser Ser
   355 360 365
   Leu Gly Leu Thr Asp Glu Glu Lys Glu Ala Ala Glu His Phe Leu Asn
   370 375 380
   Val Ser Asp Asp Ser Gln Asn Asp Tyr Glu
   385 390
<210> 7
   <211> 241
   <212> PRT
   <213> Human respiratory syncytial virus
<400> 7
   Met Glu Lys Phe Ala Pro Glu Phe His Gly Glu Asp Ala Asn Asn Arg
   1               5                   10                  15
   Ala Thr Lys Phe Leu Glu Ser Ile Lys Gly Lys Phe Thr Ser Pro Lys
               20                  25                  30
   Asp Pro Lys Lys Lys Asp Ser Ile Ile Ser Val Asn Ser Thr Asp Ile
           35                  40                  45
   Glu Val Thr Lys Glu Ser Pro Ile Thr Ser Asn Ser Thr Ile Ile Asn
       50                  55                  60
   Pro Thr Asn Glu Thr Asp Asp Asn Ala Gly Asn Lys Pro Asn Tyr Gln
   65                  70                  75                  80
   Arg Lys Pro Leu Val Ser Phe Lys Glu Asp Pro Ile Pro Ser Asp Asn
                   85                  90                  95
   Pro Phe Ser Lys Leu Tyr Lys Glu Thr Ile Glu Thr Phe Asp Asn Asn
               100                 105                 110
   Glu Glu Glu Ser Ser Tyr Ser Tyr Glu Glu Ile Asn Asp Gln Thr Asn
           115                 120                 125
   Asp Asn Ile Thr Ala Arg Leu Asp Arg Ile Asp Glu Lys Leu Ser Glu
       130                 135                 140
   Ile Leu Gly Met Leu His Thr Leu Val Val Ala Ser Ala Gly Pro Thr
   145                 150                 155                 160
   Ser Ala Arg Asp Gly Ile Arg Asp Ala Met Val Gly Leu Arg Glu Glu
                   165                 170                 175
   Met Ile Glu Lys Ile Arg Thr Glu Ala Leu Met Thr Asn Asp Arg Leu
               180                 185                 190
   Glu Ala Met Ala Arg Leu Arg Asn Glu Glu Ser Glu Lys Met Ala Lys
           195                 200                 205
   Asp Thr Ser Asp Glu Val Ser Leu Asn Pro Thr Ser Glu Lys Leu Asn
       210                 215                 220
   Asn Leu Leu Glu Gly Asn Asp Ser Asp Asn Asp Leu Ser Leu Glu Asp
   225                 230                 235                 240
   Phe
<210> 8
   <211> 241
   <212> PRT
   <213> Bovine respiratory syncytial virus
<400> 8
   Met Glu Lys Phe Ala Pro Glu Phe His Gly Glu Asp Ala Asn Thr Lys
   1               5                   10                  15
   Ala Thr Lys Phe Leu Glu Ser Leu Lys Gly Lys Phe Thr Ser Ser Lys
               20                  25                  30
   Asp Ser Arg Lys Lys Asp Ser Ile Ile Ser Val Asn Ser Ile Asp Ile
           35                  40                  45
   Glu Leu Pro Lys Glu Ser Pro Ile Thr Ser Thr Asn His Asn Ile Asn
   50 55 60
   Gln Pro Ser Glu Ile Asn Asp Thr Ile Ala Ala Asn Gln Val His Ile
   65 70 75 80
   Arg Lys Pro Leu Val Ser Phe Lys Glu Glu Leu Pro Ser Ser Glu Asn
   85 90 95
   Pro Phe Thr Lys Leu Tyr Lys Glu Thr Ile Glu Thr Phe Asp Asn Asn
   100 105 110
   Glu Glu Glu Ser Ser Tyr Ser Tyr Asp Glu Ile Asn Asp Gln Thr Asn
   115 120 125
   Asp Asn Ile Thr Ala Arg Leu Asp Arg Ile Asp Glu Lys Leu Ser Glu
   130 135 140
   Ile Ile Gly Met Leu His Thr Leu Val Val Ala Ser Ala Gly Pro Thr
   145 150 155 160
   Ala Ala Arg Asp Gly Ile Arg Asp Ala Met Val Gly Leu Arg Glu Glu
   165 170 175
   Met Ile Glu Lys Ile Arg Ser Glu Ala Leu Met Thr Asn Asp Arg Leu
   180 185 190
   Glu Ala Met Ala Arg Leu Arg Asp Glu Glu Ser Glu Lys Met Thr Lys
   195 200 205
   Asp Thr Ser Asp Glu Val Lys Leu Thr Pro Thr Ser Glu Lys Leu Asn
   210 215 220
   Met Val Leu Glu Asp Glu Ser Ser Asp Asn Asp Leu Ser Leu Glu Asp
   225 230 235 240
   Phe
<210> 9
   <211> 568
   <212> PRT
   <213> Sendai virus
<400> 9
   Met Asp Gln Asp Ala Phe Ile Leu Lys Glu Asp Ser Glu Val Glu Arg
   1 5 10 15
   Glu Ala Pro Gly Gly Arg Glu Ser Leu Ser Asp Val Ile Gly Phe Leu
               20                  25                  30
   Asp Ala Val Leu Ser Ser Glu Pro Thr Asp Ile Gly Gly Asp Arg Ser
           35                  40                  45
   Trp Leu His Asn Thr Ile Asn Thr Pro Gln Gly Pro Gly Ser Ala His
       50                  55                  60
   Arg Ala Lys Ser Glu Gly Glu Gly Glu Val Ser Thr Pro Ser Thr Gln
   65                  70                  75                  80
   Asp Asn Arg Ser Gly Glu Glu Ser Arg Val Ser Gly Arg Thr Ser Lys
                   85                  90                  95
   Pro Glu Ala Glu Ala His Ala Gly Asn Leu Asp Lys Gln Asn Ile His
               100                 105                 110
   Arg Ala Phe Gly Gly Arg Thr Gly Thr Asn Ser Val Ser Gln Asp Leu
           115                 120                 125
   Gly Asp Gly Gly Asp Ser Gly Ile Leu Glu Asn Pro Pro Asn Glu Arg
       130                 135                 140
   Gly Tyr Pro Arg Ser Gly Ile Glu Asp Glu Asn Arg Glu Met Ala Ala
   145                 150                 155                 160
   His Pro Asp Lys Arg Gly Glu Asp Gln Ala Glu Gly Leu Pro Glu Glu
                   165                 170                 175
   Val Arg Gly Gly Thr Ser Leu Pro Asp Glu Gly Glu Gly Gly Ala Ser
               180                 185                 190
   Asn Asn Gly Arg Ser Met Glu Pro Gly Ser Ser His Ser Ala Arg Val
           195                 200                 205
   Thr Gly Val Leu Val Ile Pro Ser Pro Glu Leu Glu Glu Ala Val Leu
       210                 215                 220
   Arg Arg Asn Lys Arg Arg Pro Thr Asn Ser Gly Ser Lys Pro Leu Thr
   225                 230                 235                 240
   Pro Ala Thr Val Pro Gly Thr Arg Ser Pro Pro Leu Asn Arg Tyr Asn
                   245                 250                 255
   Ser Thr Gly Ser Pro Pro Gly Lys Pro Pro Ser Thr Gln Asp Glu His
               260                 265                 270
   Ile Asn Ser Gly Asp Thr Pro Ala Val Arg Val Lys Asp Arg Lys Pro
           275                 280                 285
   Pro Ile Gly Thr Arg Ser Val Ser Asp Cys Pro Ala Asn Gly Arg Pro
       290                 295                 300
   Ile His Pro Gly Leu Glu Ser Asp Ser Thr Lys Lys Gly Ile Gly Glu
   305                 310                 315                 320
   Asn Thr Ser Ser Met Lys Glu Met Ala Thr Leu Leu Thr Ser Leu Gly
                   325                 330                 335
   Val Ile Gln Ser Ala Gln Glu Phe Glu Ser Ser Arg Asp Ala Ser Tyr
               340                 345                 350
   Val Phe Ala Arg Arg Ala Leu Lys Ser Ala Asn Tyr Ala Glu Met Thr
           355                 360                 365
   Phe Asn Val Cys Gly Leu Ile Leu Ser Ala Glu Lys Ser Ser Ala Arg
       370                 375                 380
   Lys Val Asp Glu Asn Lys Gln Leu Leu Lys Gln Ile Gln Glu Ser Val
   385                 390                 395                 400
   Glu Ser Phe Arg Asp Ile Tyr Lys Arg Phe Ser Glu Tyr Gln Lys Glu
                   405                 410                 415
   Gln Asn Ser Leu Leu Met Ser Asn Leu Ser Thr Leu His Ile Ile Thr
               420                 425                 430
   Asp Arg Gly Gly Lys Thr Asp Asn Thr Asp Ser Leu Thr Arg Ser Pro
           435                 440                 445
   Ser Val Phe Ala Lys Ser Lys Glu Asn Lys Thr Lys Ala Thr Arg Phe
       450                 455                 460
   Asp Pro Ser Met Glu Thr Leu Glu Asp Met Lys Tyr Lys Pro Asp Leu
   465                 470                 475                 480
   Ile Arg Glu Asp Glu Phe Arg Asp Glu Ile Arg Asn Pro Val Tyr Gln
                   485                 490                 495
   Glu Arg Asp Thr Glu Pro Arg Ala Ser Asn Ala Ser Arg Leu Leu Pro
   500 505 510
   Ser Lys Glu Lys Pro Thr Met His Ser Leu Arg Leu Val Ile Glu Ser
   515 520 525
   Ser Pro Leu Ser Arg Ala Glu Lys Ala Ala Tyr Val Lys Ser Leu Ser
   530 535 540
   Lys Cys Lys Thr Asp Gln Glu Val Lys Ala Val Met Glu Leu Val Glu
   545 550 555 560
   Glu Asp Ile Glu Ser Leu Thr Asn
   565
<210> 10
   <211> 507
   <212> PRT
   <213> Measles virus
<400> 10
   Met Ala Glu Glu Gln Ala Arg His Val Lys Asn Gly Leu Glu Cys Ile
   1 5 10 15
   Arg Ala Leu Lys Ala Glu Pro Ile Gly Ser Leu Ala Val Glu Glu Ala
   20 25 30
   Met Ala Ala Trp Ser Glu Ile Ser Asp Asn Pro Gly Gln Asp Arg Ala
   35 40 45
   Thr Cys Lys Glu Glu Glu Ala Gly Ser Ser Gly Leu Ser Lys Pro Cys
   50 55 60
   Leu Ser Ala Ile Gly Ser Thr Glu Gly Gly Ala Pro Arg Ile Arg Gly
   65 70 75 80
   Gln Gly Ser Gly Glu Ser Asp Asp Asp Ala Glu Thr Leu Gly Ile Pro
   85 90 95
   Ser Arg Asn Leu Gln Ala Ser Ser Thr Gly Leu Gln Cys Tyr His Val
   100 105 110
   Tyr Asp His Ser Gly Glu Ala Val Lys Gly Ile Gln Asp Ala Asp Ser
   115 120 125
   Ile Met Val Gln Ser Gly Leu Asp Gly Asp Ser Thr Leu Ser Gly Gly
   130 135 140
   Asp Asp Glu Ser Glu Asn Ser Asp Val Asp Ile Gly Glu Pro Asp Thr
   145                 150                 155                 160
   Glu Gly Tyr Ala Ile Thr Asp Arg Gly Ser Ala Pro Ile Ser Met Gly
                   165                 170                 175
   Phe Arg Ala Ser Asp Val Glu Thr Ala Glu Gly Gly Glu Ile His Glu
               180                 185                 190
   Leu Leu Lys Leu Gln Ser Arg Gly Asn Asn Phe Pro Lys Leu Gly Lys
           195                 200                 205
   Thr Leu Asn Val Pro Pro Pro Pro Asn Pro Ser Arg Ala Ser Thr Ser
       210                 215                 220
   Glu Thr Pro Ile Lys Lys Gly Thr Asp Ala Arg Leu Ala Ser Phe Gly
   225                 230                 235                 240
   Thr Glu Ile Ala Ser Leu Leu Thr Gly Gly Ala Thr Gln Cys Ala Arg
                   245                 250                 255
   Lys Ser Pro Ser Glu Pro Ser Gly Pro Gly Ala Pro Ala Gly Asn Val
               260                 265                 270
   Pro Glu Cys Val Ser Asn Ala Ala Leu Ile Gln Glu Trp Thr Pro Glu
           275                 280                 285
   Ser Gly Thr Thr Ile Ser Pro Arg Ser Gln Asn Asn Glu Glu Gly Gly
       290                 295                 300
   Asp Tyr Tyr Asp Asp Glu Leu Phe Ser Asp Val Gln Asp Ile Lys Thr
   305                 310                 315                 320
   Ala Leu Ala Lys Ile His Glu Asp Asn Gln Lys Ile Ile Ser Lys Leu
                   325                 330                 335
   Glu Ser Leu Leu Leu Leu Lys Gly Glu Val Glu Ser Ile Lys Lys Gln
               340                 345                 350
   Ile Asn Arg Gln Asn Ile Ser Ile Ser Thr Leu Glu Gly His Leu Ser
           355                 360                 365
   Ser Ile Met Ile Ala Ile Pro Gly Leu Gly Lys Asp Pro Asn Asp Pro
       370                 375                 380
   Thr Ala Asp Val Glu Leu Asn Pro Asp Leu Lys Pro Ile Ile Gly Arg
   385 390 395 400
   Asp Ser Gly Arg Ala Leu Ala Glu Val Leu Lys Lys Pro Val Ala Ser
   405 410 415
   Arg Gln Leu Gln Gly Met Thr Asn Gly Arg Thr Ser Ser Arg Gly Gln
   420 425 430
   Leu Leu Lys Glu Phe Gln Leu Lys Pro Ile Gly Lys Lys Val Ser Ser
   435 440 445
   Ala Val Gly Phe Val Pro Asp Thr Gly Pro Ala Ser Arg Ser Val Ile
   450 455 460
   Arg Ser Ile Ile Lys Ser Ser Arg Leu Glu Glu Asp Arg Lys Arg Tyr
   465 470 475 480
   Leu Met Thr Leu Leu Asp Asp Ile Lys Gly Ala Asn Asp Leu Ala Lys
   485 490 495
   Phe His Gln Met Leu Met Lys Ile Ile Met Lys
   500 505
<210> 11
   <211> 390
   <212> PRT
   <213> Mumps virus
<400> 11
   Met Asp Gln Phe Ile Lys Gln Asp Glu Thr Gly Asp Leu Ile Glu Thr
   1               5                   10                  15
   Gly Met Asn Val Ala Asn His Phe Leu Ser Ala Pro Ile Gln Gly Thr
               20                  25                  30
   Asn Ser Leu Ser Lys Ala Ser Ile Ile Pro Gly Val Ala Pro Val Leu
           35                  40                  45
   Ile Gly Asn Pro Glu Gln Lys Asn Ile Gln His Pro Thr Ala Ser His
       50                  55                  60
   Gln Gly Ser Lys Ser Lys Gly Ser Gly Ser Gly Val Arg Ser Ile Ile
   65                  70                  75                  80
   Val Pro Pro Ser Glu Ala Ser Asn Gly Gly Thr Gln Ile Pro Glu Pro
                   85                  90                  95
   Leu Phe Ala Gln Thr Gly Gln Gly Gly Ile Val Thr Thr Val Tyr Gln
               100                 105                 110
   Asp Pro Thr Ile Gln Pro Thr Gly Ser Tyr Arg Ser Val Glu Leu Ala
           115                 120                 125
   Lys Ile Gly Lys Glu Arg Met Ile Asn Arg Phe Val Glu Lys Pro Arg
       130                 135                 140
   Thr Ser Thr Pro Val Thr Glu Phe Lys Arg Gly Ala Gly Ser Arg Ala
   145                 150                 155                 160
   Gln Gly Gln Thr Ile Gln Glu Glu Gly Ile Asp Gly Asn Gly Ala Ser
                   165                 170                 175
   Ala Gly Ser Lys Glu Arg Ser Gly Ser Leu Ser Gly Ala Thr Leu Tyr
               180                 185                 190
   Ala His Leu Ser Leu Pro Gln Gln Asp Ser Thr Pro Ala Asn Val Gly
           195                 200                 205
   Ile Ala Pro Gln Ser Ala Ile Ser Ala Asn Glu Ile Met Asp Leu Leu
       210                 215                 220
   Arg Gly Met Asp Ala Arg Leu Gln His Leu Glu Gln Lys Val Asp Lys
   225                 230                 235                 240
   Val Leu Ala Gln Gly Ser Met Val Thr Gln Ile Lys Asn Glu Leu Ser
                   245                 250                 255
   Thr Val Lys Thr Thr Leu Ala Thr Ile Glu Gly Met Met Ala Thr Val
               260                 265                 270
   Lys Ile Met Asp Pro Gly Asn Pro Thr Gly Val Pro Val Asp Glu Leu
           275                 280                 285
   Arg Arg Ser Phe Ser Asp His Val Thr Ile Val Ser Gly Pro Gly Asp
       290                 295                 300
   Val Pro Phe Ser Ser Ser Glu Glu Pro Thr Leu Tyr Leu Asp Glu Leu
   305                 310                 315                 320
   Ala Arg Pro Val Ser Lys Pro Arg Pro Ala Lys Gln Thr Lys Pro Gln
                   325                 330                 335
   Pro Val Lys Asp Leu Ala Gly Arg Lys Val Met Ile Thr Lys Met Ile
                   340                 345                 350
   Thr Asp Cys Val Ala Asn Pro Gln Met Lys Gln Ala Phe Glu Gln Arg
               355                 360                 365
   Leu Ala Lys Ala Ser Thr Glu Asp Ala Leu Asn Asp Ile Lys Lys Asp
       370                 375                 380
   Ile Ile Arg Ser Ala Ile
   385                 390
<210> 12
   <211> 294
   <212> PRT
   <213> Human Metapneumovirus
<400> 12
   Met Ser Phe Pro Glu Gly Lys Asp Ile Leu Phe Met Gly Asn Glu Ala
   1               5                   10                  15
   Ala Lys Leu Ala Glu Ala Phe Gln Lys Ser Leu Arg Lys Pro Gly His
               20                  25                  30
   Lys Arg Ser Gln Ser Ile Ile Gly Glu Lys Val Asn Thr Val Ser Glu
           35                  40                  45
   Thr Leu Glu Leu Pro Thr Ile Ser Arg Pro Ala Lys Pro Thr Ile Pro
       50                  55                  60
   Ser Glu Pro Lys Leu Ala Trp Thr Asp Lys Gly Gly Ala Thr Lys Thr
   65                  70                  75                  80
   Glu Ile Lys Gln Ala Ile Lys Val Met Asp Pro Ile Glu Glu Glu Glu
                   85                  90                  95
   Ser Thr Glu Lys Lys Val Leu Pro Ser Ser Asp Gly Lys Thr Pro Ala
               100                 105                 110
   Glu Lys Lys Leu Lys Pro Ser Thr Asn Thr Lys Lys Lys Val Ser Phe
           115                 120                 125
   Thr Pro Asn Glu Pro Gly Lys Tyr Thr Lys Leu Glu Lys Asp Ala Leu
       130                 135                 140
   Asp Leu Leu Ser Asp Asn Glu Glu Glu Asp Ala Glu Ser Ser Ile Leu
   145 150 155 160
   Thr Phe Glu Glu Arg Asp Thr Ser Ser Leu Ser Ile Glu Ala Arg Leu
   165 170 175
   Glu Ser Ile Glu Glu Lys Leu Ser Met Ile Leu Gly Leu Leu Arg Thr
   180 185 190
   Leu Asn Ile Ala Thr Ala Gly Pro Thr Ala Ala Arg Asp Gly Ile Arg
   195 200 205
   Asp Ala Met Ile Gly Val Arg Glu Glu Leu Ile Ala Asp Ile Ile Lys
   210 215 220
   Glu Ala Lys Gly Lys Ala Ala Glu Met Met Glu Glu Glu Met Ser Gln
   225 230 235 240
   Arg Ser Lys Ile Gly Asn Gly Ser Val Lys Leu Thr Glu Lys Ala Lys
   245 250 255
   Glu Leu Asn Lys Ile Val Glu Asp Glu Ser Thr Ser Gly Glu Ser Glu
   260 265 270
   Glu Glu Glu Glu Pro Lys Asp Thr Gln Asp Asn Ser Gln Glu Asp Asp
   275 280 285
   Ile Tyr Gln Leu Ile Met
   290
<210> 13
   <211> 31
   <212> DNA
   <213> Artificial
<220>
   <223> primer
<400> 13
   gagggatcca tcatggaaaa gtttgctcct g 31
<210> 14
   <211> 27
   <212> DNA
   <213> Artificial
<220>
   <223> primer
<400> 14
   ctgttggtgt tgtgtgttga agtgcag 27
<210> 15
   <211> 31
   <212> DNA
   <213> Artificial
<220>
   <223> primer
<400> 15
   gagggatcct ctgctaggga tggtataaga g 31
<210> 16
   <211> 36
   <212> DNA
   <213> Artificial
<220>
   <223> primer
<400> 16
   gagggatcca aaatcagaac tgaagcatta atgacc 36
<210> 17
   <211> 34
   <212> DNA
   <213> Artificial
<220>
   <223> primer
<400> 17
   gagggatccg aggaaagtga aaagatggca aaag 34
<210> 18
   <211> 31
   <212> DNA
   <213> Artificiel
<220>
   <223> primer
<400> 18
   gagggatccg agaaattgaa caacctgttg g 31
<210> 19
   <211> 44
   <212> DNA
   <213> Artificial
<220>
   <223> primer
<400> 19
   gatccaatga tagtgacaat gatctatcac ttgaagattt ctga 44
<210> 20
   <211> 40
   <212> DNA
   <213> Artificial
<220>
   <223> primer
<400> 20
   tcagaaatct tcaagtgata gatcattgtc actatcattg 40
<210> 21
   <211> 33
   <212> DNA
   <213> Artificial
<220>
   <223> primer
<400> 21
   gagggatcca tggctcttag caaagtcaag ttg 33
<210> 22
   <211> 32
   <212> DNA
   <213> Artificial
<220>
   <223> primer
<400> 22
   ttaactcaaa gctctacatc attatctttt gg 32
<210> 23
   <211> 43
   <212> DNA
   <213> Artificial
<220>
   <223> primer
<400> 23
   ccaaaagata atgatgtaga gctctgactc gagcaccacc acc 43
<210> 24
   <211> 43
   <212> DNA
   <213> Artificial
<220>
   <223> primer
<400> 24
   ggtggtggtg ctcgagtcag agctctacat cattatcttt tgg 43
<210> 25
   <211> 55
   <212> DNA
   <213> Artificial
<220>
   <223> epitope
<400> 25
   catggactac aaggacgacg atgacaagca ctggtctatc tctaaaccgc agtag 55
<210> 26
   <211> 63
   <212> DNA
   <213> Artificial
<220>
   <223> epitope
<400> 26
   tcgactactg cggtttagag atagaccagt gcttgtcatc gtcgtccttg tagtccatga 60
   gct 63
<210> 27
   <211> 40
   <212> DNA
   <213> Artificial
<220>
   <223> epitope
<400> 27
   ccggtctcta ctaacaaagc tgttgtttct ctgagctagt 40
<210> 28
   <211> 10
   <212> PRT
   <213> Artificial
<220>
   <223> epitope
<400> 28
   Ser Thr Asn Lys Ala Val Val Ser Leu Ser
   1 5 10
<210> 29
   <211> 40
   <212> DNA
   <213> Artificial
<220>
   <223> epitope
<400> 29
   ccggactagc tcagagaaac aacagctttg ttagtagaga 40
<210> 30
   <211> 41
   <212> DNA
   <213> Artificial
<220>
   <223> primer
<400> 30
   gatccgatag tgacaatgat ctatcacttg aagatttctg a 41
<210> 31
   <211> 37
   <212> DNA
   <213> Artificial
<220>
   <223> primer
<400> 31
   tcagaaatct tcaagtgata gatcattgtc actatcg 37
<210> 32
   <211> 32
   <212> PRT
   <213> Artificial
<220>
   <223> linker
<400> 32
   Lys Leu Arg Ile Leu Gln Ser Thr Val Pro Ser Glu Arg Pro Gln Ala
   1               5                   10                  15
   Ser Gly Val Tyr Met Gly Asn Leu Thr Thr Arg Gly Pro Val Ala Thr
               20                  25                  30
<210> 33
   <211> 667
   <212> PRT
   <213> Artificial
<220>
   <223> N-GFP fusion protein
<400> 33
   Gly Ser His Met Ala Ser Met Thr Gly Gly Gln Gln Met Gly Arg Gly
   1               5                   10                  15
   Ser Met Ala Leu Ser Lys Val Lys Leu Asn Asp Thr Leu Asn Lys Asp
               20                  25                  30
   Gln Leu Leu Ser Ser Ser Lys Tyr Thr Ile Gln Arg Ser Thr Gly Asp
   35 40 45
   Ser Ile Asp Thr Pro Asn Tyr Asp Val Gln Lys His Ile Asn Lys Leu
   50 55 60
   Cys Gly Met Leu Leu Ile Thr Glu Asp Ala Asn His Lys Phe Thr Gly
   65 70 75 80
   Leu Ile Gly Met Leu Tyr Ala Met Ser Arg Leu Gly Arg Glu Asp Thr
   85 90 95
   Ile Lys Ile Leu Arg Asp Ala Gly Tyr His Val Lys Ala Asn Gly Val
   100 105 110
   Asp Val Thr Thr His Arg Gln Asp Ile Asn Gly Lys Glu Met Lys Phe
   115 120 125
   Glu Val Leu Thr Leu Ser Ser Leu Thr Thr Glu Ile Gln Ile Asn Ile
   130 135 140
   Glu Ile Glu Ser Arg Lys Ser Tyr Lys Lys Met Leu Lys Glu Met Gly
   145 150 155 160
   Glu Val Ala Pro Glu Tyr Arg His Asp Ser Pro Asp Cys Gly Met Ile
   165 170 175
   Ile Leu Cys Ile Ala Ala Leu Val Ile Thr Lys Leu Ala Ala Gly Asp
   180 185 190
   Arg Ser Gly Leu Thr Ala Val Ile Arg Arg Ala Asn Asn Val Leu Lys
   195 200 205
   Asn Glu Met Lys Arg Tyr Lys Gly Leu Leu Pro Lys Asp Ile Ala Asn
   210 215 220
   Ser Phe Tyr Glu Val Phe Glu Lys Tyr Pro His Phe Ile Asp Val Phe
   225 230 235 240
   Val His Phe Gly Ile Ala Gln Ser Ser Thr Arg Gly Gly Ser Arg Val
   245 250 255
   Glu Gly Ile Phe Ala Gly Leu Phe Met Asn Ala Tyr Gly Ala Gly Gln
   260 265 270
   Val Met Leu Arg Trp Gly Val Leu Ala Lys Ser Val Lys Asn Ile Met
   275 280 285
   Leu Gly His Ala Ser Val Gln Ala Glu Met Glu Gln Val Val Glu Val
   290 295 300
   Tyr Glu Tyr Ala Gln Lys Leu Gly Gly Glu Ala Gly Phe Tyr His Ile
   305 310 315 320
   Leu Asn Asn Pro Lys Ala Ser Leu Leu Ser Leu Thr Gln Phe Pro His
   325 330 335
   Phe Ser Ser Val Val Leu Gly Asn Ala Ala Gly Leu Gly Ile Met Gly
   340 345 350
   Glu Tyr Arg Gly Thr Pro Arg Asn Gln Asp Leu Tyr Asp Ala Ala Lys
   355 360 365
   Ala Tyr Ala Glu Gln Leu Lys Glu Asn Gly Val Ile Asn Tyr Ser Val
   370 375 380
   Leu Asp Leu Thr Ala Glu Glu Leu Glu Ala Ile Lys His Gln Leu Asn
   385 390 395 400
   Pro Lys Asp Asn Asp Val Leu Glu Leu Lys Leu Arg Ile Leu Gln Ser
   405 410 415
   Thr Val Pro Arg Ala Arg Asp Pro Pro Val Ala Thr Met Val Ser Lys
   420 425 430
   Gly Glu Glu Leu Phe Thr Gly Val Val Pro Ile Leu Val Glu Leu Asp
   435 440 445
   Gly Asp Val Asn Gly His Lys Phe Ser Val Ser Gly Glu Gly Glu Gly
   450 455 460
   Asp Ala Thr Tyr Gly Lys Leu Thr Leu Lys Phe Ile Cys Thr Thr Gly
   465 470 475 480
   Lys Leu Pro Val Pro Trp Pro Thr Leu Val Thr Thr Leu Thr Tyr Gly
   485 490 495
   Val Gln Cys Phe Ser Arg Tyr Pro Asp His Met Lys Gln His Asp Phe
   500 505 510
   Phe Lys Ser Ala Met Pro Glu Gly Tyr Val Gln Glu Arg Thr Ile Phe
   515 520 525
   Phe Lys Asp Asp Gly Asn Tyr Lys Thr Arg Ala Glu Val Lys Phe Glu
   530 535 540
   Gly Asp Thr Leu Val Asn Arg Ile Glu Leu Lys Gly Ile Asp Phe Lys
   545 550 555 560
   Glu Asp Gly Asn Ile Leu Gly His Lys Leu Glu Tyr Asn Tyr Asn Ser
   565 570 575
   His Asn Val Tyr Ile Met Ala Asp Lys Gln Lys Asn Gly Ile Lys Val
   580 585 590
   Asn Phe Lys Ile Arg His Asn Ile Glu Asp Gly Ser Val Gln Leu Ala
   595 600 605
   Asp His Tyr Gln Gln Asn Thr Pro Ile Gly Asp Gly Pro Val Leu Leu
   610 615 620
   Pro Asp Asn His Tyr Leu Ser Thr Gln Ser Ala Leu Ser Lys Asp Pro
   625 630 635 640
   Asn Glu Lys Arg Asp His Met Val Leu Leu Glu Phe Val Thr Ala Ala
   645 650 655
   Gly Ile Thr Leu Gly Met Asp Glu Leu Tyr Lys
   660 665
<210> 34
   <211> 19
   <212> PRT
   <213> Artificial
<220>
   <223> linker
<400> 34
   Lys Leu Arg Ile Leu Gln Ser Thr Val Pro Arg Ala Arg Asp Pro Pro
   1 5 10 15
   Val Ala Thr
<210> 35
   <211> 680
   <212> PRT
   <213> Artificial
<220>
   <223> N-GFP fusion protein
<400> 35
   Gly Ser His Met Ala Ser Met Thr Gly Gly Gln Gln Met Gly Arg Gly
   1               5                   10                  15
   Ser Met Ala Leu Ser Lys Val Lys Leu Asn Asp Thr Leu Asn Lys Asp
               20                  25                  30
   Gln Leu Leu Ser Ser Ser Lys Tyr Thr Ile Gln Arg Ser Thr Gly Asp
           35                  40                  45
   Ser Ile Asp Thr Pro Asn Tyr Asp Val Gln Lys His Ile Asn Lys Leu
       50                  55                  60
   Cys Gly Met Leu Leu Ile Thr Glu Asp Ala Asn His Lys Phe Thr Gly
   65                  70                  75                  80
   Leu Ile Gly Met Leu Tyr Ala Met Ser Arg Leu Gly Arg Glu Asp Thr
                   85                  90                  95
   Ile Lys Ile Leu Arg Asp Ala Gly Tyr His Val Lys Ala Asn Gly Val
               100                 105                 110
   Asp Val Thr Thr His Arg Gln Asp Ile Asn Gly Lys Glu Met Lys Phe
           115                 120                 125
   Glu Val Leu Thr Leu Ser Ser Leu Thr Thr Glu Ile Gln Ile Asn Ile
       130                 135                 140
   Glu Ile Glu Ser Arg Lys Ser Tyr Lys Lys Met Leu Lys Glu Met Gly
   145                 150                 155                 160
   Glu Val Ala Pro Glu Tyr Arg His Asp Ser Pro Asp Cys Gly Met Ile
                   165                 170                 175
   Ile Leu Cys Ile Ala Ala Leu Val Ile Thr Lys Leu Ala Ala Gly Asp
               180                 185                 190
   Arg Ser Gly Leu Thr Ala Val Ile Arg Arg Ala Asn Asn Val Leu Lys
           195                 200                 205
   Asn Glu Met Lys Arg Tyr Lys Gly Leu Leu Pro Lys Asp Ile Ala Asn
       210                 215                 220
   Ser Phe Tyr Glu Val Phe Glu Lys Tyr Pro His Phe Ile Asp Val Phe
   225 230 235 240
   Val His Phe Gly Ile Ala Gln Ser Ser Thr Arg Gly Gly Ser Arg Val
   245 250 255
   Glu Gly Ile Phe Ala Gly Leu Phe Met Asn Ala Tyr Gly Ala Gly Gln
   260 265 270
   Val Met Leu Arg Trp Gly Val Leu Ala Lys Ser Val Lys Asn Ile Met
   275 280 285
   Leu Gly His Ala Ser Val Gln Ala Glu Met Glu Gln Val Val Glu Val
   290 295 300
   Tyr Glu Tyr Ala Gln Lys Leu Gly Gly Glu Ala Gly Phe Tyr His Ile
   305 310 315 320
   Leu Asn Asn Pro Lys Ala Ser Leu Leu Ser Leu Thr Gln Phe Pro His
   325 330 335
   Phe Ser Ser Val Val Leu Gly Asn Ala Ala Gly Leu Gly Ile Met Gly
   340 345 350
   Glu Tyr Arg Gly Thr Pro Arg Asn Gln Asp Leu Tyr Asp Ala Ala Lys
   355 360 365
   Ala Tyr Ala Glu Gln Leu Lys Glu Asn Gly Val Ile Asn Tyr Ser Val
   370 375 380
   Leu Asp Leu Thr Ala Glu Glu Leu Glu Ala Ile Lys His Gln Leu Asn
   385 390 395 400
   Pro Lys Asp Asn Asp Val Leu Glu Leu Lys Leu Arg Ile Leu Gln Ser
   405 410 415
   Thr Val Pro Ser Glu Arg Pro Gln Ala Ser Gly Val Tyr Met Gly Asn
   420 425 430
   Leu Thr Thr Arg Gly Pro Val Ala Thr Met Val Ser Lys Gly Glu Glu
   435 440 445
   Leu Phe Thr Gly Val Val Pro Ile Leu Val Glu Leu Asp Gly Asp Val
   450 455 460
   Asn Gly His Lys Phe Ser Val Ser Gly Glu Gly Glu Gly Asp Ala Thr
   465                 470                 475                 480
   Tyr Gly Lys Leu Thr Leu Lys Phe Ile Cys Thr Thr Gly Lys Leu Pro
                   485                 490                 495
   Val Pro Trp Pro Thr Leu Val Thr Thr Leu Thr Tyr Gly Val Gln Cys
               500                 505                 510
   Phe Ser Arg Tyr Pro Asp His Met Lys Gln His Asp Phe Phe Lys Ser
           515                 520                 525
   Ala Met Pro Glu Gly Tyr Val Gln Glu Arg Thr Ile Phe Phe Lys Asp
       530                 535                 540
   Asp Gly Asn Tyr Lys Thr Arg Ala Glu Val Lys Phe Glu Gly Asp Thr
   545                 550                 555                 560
   Leu Val Asn Arg Ile Glu Leu Lys Gly Ile Asp Phe Lys Glu Asp Gly
                   565                 570                 575
   Asn Ile Leu Gly His Lys Leu Glu Tyr Asn Tyr Asn Ser His Asn Val
               580                 585                 590
   Tyr Ile Met Ala Asp Lys Gln Lys Asn Gly Ile Lys Val Asn Phe Lys
           595                 600                 605
   Ile Arg His Asn Ile Glu Asp Gly Ser Val Gln Leu Ala Asp His Tyr
       610                 615                 620
   Gln Gln Asn Thr Pro Ile Gly Asp Gly Pro Val Leu Leu Pro Asp Asn
   625                 630                 635                 640
   His Tyr Leu Ser Thr Gln Ser Ala Leu Ser Lys Asp Pro Asn Glu Lys
                   645                 650                 655
   Arg Asp His Met Val Leu Leu Glu Phe Val Thr Ala Ala Gly Ile Thr
               660                 665                 670
   Leu Gly Met Asp Glu Leu Tyr Lys
           675                 680

## Description

La demande décrit des protéines de fusion protéine N-protéine d'intérêt, éventuellement sous forme de complexes solubles protéine N-protéine d'intérêt/protéine P, les protéines N et P étant des protéines d'un virus de la famille des *Paramyxoviridae.* Lorsque la protéine d'intérêt est un antigène, la demande décrit également des compositions vaccinales et des réactifs de diagnostic comprenant ces protéines de fusion protéine N-antigène ou ces complexes protéine N-antigène/protéine P. La fusion protéine N-protéine d'intérêt peut également être utilisée comme « vecteur » pour transporter, dans les cellules, des molécules d'intérêt thérapeutiques tels que des antiviraux ou des anticancéreux.

Le virus respiratoire syncytial (RSV selon la dénomination internationale) est responsable de la bronchiolite du nourrisson (500 000 enfants atteints chaque année en France). Il n'existe aucun traitement préventif (vaccin) ni d'antiviral réellement efficace contre cette maladie. La même maladie existe chez les bovins, atteignant 70% des veaux la première année, la mortalité pouvant atteindre 20%, et le RSV bovin est le principal agent responsable de maladies respiratoires graves chez le veau. Le tableau clinique est identique à celui de l'homme. Ce virus appartient à l'ordre des *Mononegavirales,* à la famille des *Paramyxoviridae.* Les particules virales sont entourées par une enveloppe lipidique contenant deux protéines majeures, la protéine de fusion (F) et la glycoprotéine (G). A l'intérieur des particules, on trouve un ARN simple brin de polarité négative d'environ 15 kb, associé à la protéine de nucléocapside (N). Ce complexe ARN-N constitue la matrice du complexe polymérase constitué de la protéine L (large fragment) qui est l'ARN polymérase ARN-dépendante, et son co-facteur P (phosphoprotéine) également présents dans les virions.

Jusqu'alors, il n'était pas possible de purifier la protéine N sous forme soluble, car exprimée sous forme recombinante, elle s'associe spontanément et de façon non spécifique aux ARNs cellulaires, formant des structures de très haut poids moléculaire et non solubles (Méric et al. 1994 Virus Res. 31(2):187-201 ; Bhella et al., 2002 Journal of General Virology; 83, 1831-1839). Les inventeurs ont mis au point un procédé d'obtention de la protéine de nucléocapside (N) du virus respiratoire syncytial (RSV) sous forme recombinante soluble ayant une structure en anneaux et son utilisation en tant que vaccin. La protéine N se présente sous la forme d'anneaux très réguliers d'environ 7 nm de diamètre, contenant 10 molécules de protéine N et un ARN d'origine bactérienne d'environ 70 bases. La protéine N est co-exprimée chez *E. coli* avec la phosphoprotéine (P) du RSV, plus particulièrement sa partie C-terminale, fusionnée à la glutathion-S-transferase (GST), qui permet de purifier les complexes par affinité avec des billes de sepharose-glutathion. Ces structures en anneaux induisent une forte réponse immunitaire chez la souris, notamment par administration intra-nasale, y compris en l'absence d'adjuvant. Ce procédé a fait l'objet de la demande de brevet FR 2885139.

Les Inventeurs ont maintenant mis en évidence qu'il est possible d'utiliser ces structures en anneaux en tant que vecteur vaccinal. La faisabilité de cette technologie a été démontrée en fusionnant la protéine N à une autre protéine, la GFP (green fluorescent protein) par construction plasmidique. Elle a été co-exprimée chez *E. coli* avec la partie C-terminale de la protéine P (acides aminés 161-241) fusionnée à la GST. L'analyse des structures purifiées au microscope électronique a montré que le greffage de la GFP en C-terminal de la protéine N permet encore à la protéine N d'être produite sous forme d'anneaux ayant un diamètre d'environ 10 nm. Les protéines de fusion N-GFP sont fluorescentes.

Les anneaux N-ARN étant extrêmement immunogènes, les Inventeurs ont injecté ces structures à des souris afin de voir si elles pouvaient induire une forte réponse anticorps, notamment contre la GFP. Les réponses ont été comparées avec celles obtenues contre de la protéine GFP « normale » également purifiée sous forme recombinante à partir d'*E*. *coli.* Les résultats obtenus ont montré une réponse contre la GFP nettement plus forte (40 fois environ pour la première expérience) lorsqu'on utilise les structures en anneaux.

La possibilité de fusionner une protéine d'intérêt à la protéine N et de purifier les complexes sous forme d'anneaux solubles à une échelle industrielle pour un coût modéré (notamment chez *E. coli*) pourra ainsi permettre de développer des vaccins contre tout type d'épitope peu ou pas immunogène lorsqu'il est injecté seul.

Les Inventeurs ont en outre mis en évidence que ces structures protéine N-GFP/protéine P s'adsorbent et sont internalisées efficacement par différents types cellulaires. Ces résultats mettent donc en lumière l'intérêt de ces fusions protéine N-protéine d'intérêt, éventuellement sous forme de complexe protéine N-protéine d'intérêt/protéine P, en tant que vecteur d'antigène en vaccination, ou plus généralement de molécules d'intérêt thérapeutique.

L'invention concerne un procédé de préparation d'un complexe protéine N-protéine d'intérêt/protéine P, les protéines N et P étant des protéines d'un virus respiratoire syncytial (RSV), ledit procédé comprenant les étapes consistant à :
a) coexprimer une protéine de fusion protéine N d'un virus respiratoire syncytial - protéine d'intérêt, avec une protéine P tronquée du même virus respiratoire syncytial, dans laquelle la protéine d'intérêt est fusionnée en phase à l'extrémité C-terminale de la protéine N, la protéine N étant une protéine native ou une protéine N modifiée dans la région définie par les 25 derniers acides aminés C-terminaux, ladite protéine N conservant la capacité d'interagir avec la protéine P, la protéine P tronquée ne contenant pas le domaine d'oligomérisation P et comprenant un domaine de liaison à la protéine N;
b) recueillir les complexes protéine N-protéine d'intérêt/protéine P ainsi formés.

L'invention concerne aussi un complexe protéine N-protéine d'intérêt/protéine P sous forme d'anneaux solubles comprenant la protéine de fusion protéine N-protéine d'intérêt avec leur ARN, les protéines N et P étant des protéines d'un virus respiratoire syncytial, dans lequel la protéine d'intérêt est fusionnée en phase en C-terminal de la protéine N, la protéine N étant une protéine native ou une protéine N modifiée dans la région définie par les 25 derniers acides aminés C-terminaux, ladite protéine N conservant la capacité d'interagir avec la protéine P, la protéine P étant une protéine P tronquée du même virus respiratoire syncytial ne contenant pas le domaine d'oligomérisation P et comprenant un domaine de liaison à la protéine N.

L'invention concerne en outre un procédé de préparation d'une protéine N d'un virus respiratoire syncytial, fusionnée en phase à son extrémité C-terminale avec une protéine d'intérêt, ledit procédé comprenant la séparation de la protéine de fusion protéine N-protéine d'intérêt à partir du complexe protéine N-protéine d'intérêt/protéine P selon l'invention.

L'invention concerne également une protéine de fusion protéine N-protéine d'intérêt, isolée, structurée en anneaux solubles comprenant la protéine de fusion protéine N-protéine d'intérêt avec leur ARN, susceptible d'être obtenue par le procédé de préparation selon l'invention, où la protéine N est une protéine N d'un virus respiratoire syncytial et la protéine d'intérêt est fusionnée en phase, à l'extrémité C-terminale de la protéine N.

L'invention concerne une composition pharmaceutique comprenant une protéine N d'un virus respiratoire syncytial fusionnée avec une protéine d'intérêt ou un complexe protéine N-protéine d'intérêt/protéine P selon l'invention, et un véhicule pharmaceutiquement acceptable.

L'invention concerne un réactif diagnostic comprenant une protéine de fusion selon l'invention, dans laquelle la protéine d'intérêt est un antigène.

L'invention concerne également l'utilisation d'une protéine de fusion selon l'invention comprenant un antigène fusionné en phase à l'extrémité C-terminale d'une protéine N d'un virus respiratoire syncytial pour la détection *in vitro* d'anticorps dirigés contre ledit antigène de la protéine de fusion.

L'invention concerne en outre une méthode de détection d'anticorps spécifiques d'un antigène, dans un échantillon biologique, comprenant les étapes consistant à :
a) mettre en contact ledit échantillon biologique avec une protéine de fusion selon l'invention comprenant un antigène fusionné en phase en C-terminal d'une protéine N d'un virus respiratoire syncytial,
b) détecter les complexes protéine de fusion N-antigène/anticorps formés, la présence de tels complexes étant révélatrice de la présence d'anticorps spécifiques de l'antigène dans l'échantillon biologique.

### Définitions

La famille des « *Paramyxoviridae* » regroupe les sous-familles des *Paramyxovirinae* et des *Pneumovirinae.* Les *Paramyxovirinae* incluent les gènes *Respirovirus,* dont le virus prototype est le virus de Sendai, *Rubulavirus* (notamment le virus des oreillons), *Morbilivirus,* tels que le virus de la rougeole. Chacun des genres *Respirovirus* et *Rubulavirus* regroupe des souches du virus parainfluenza. La sous-famille des *Pneumovirinae* regroupe deux genres, les *Pneumovirus* et les *Metapneumovirus,* ce dernier incluant le *Metapneumovirus* humain. Le virus respiratoire syncytial (RSV) humain constitue le virus prototype du genre *Pneumovirus* appartenant à la sous-famille des *Pneumovirinae.* Les *Pneumovirus* incluent également les souches bovine et murine du RSV.

Sans autre précision, par « virus respiratoire syncytial » on entend d'une manière générale le RSV, quelle que soit la forme (humaine, bovine...), le sous-groupe (par exemple les sous-groupes A, B et S identifiés chez le RSV humain) ou la souche considérée.

Par « protéine N » on désigne la protéine de nucléocapside des *Paramyxoviridae* qui forme des structures hélicoïdales pour entourer le génome viral. La protéine N du RSV humain souche Long présente une séquence de 391 acides aminés qui est décrite par SEQ ID NO:1. La protéine N du RSV bovin comporte également 391 acides aminés (voir SEQ ID NO: 2). Une protéine N du virus de Sendai (souche Hamamatsu), du virus de la rougeole (Souche Edmonston B), du virus des oreillons (souche SBL-1), et du *Metapneumovirus* humain (souche 00-1) sont également décrites dans la base de données Swissprot sous les numéros d'accès Q9DUE3 (SEQ ID NO:3), Q89933 (SEQ ID NO:4), P21277 (SEQ ID NO:5), et Q91 F57 (SEQ ID NO:6), respectivement.

L'expression « protéine P » désigne la Phosphoprotéine ou protéine P faisant partie du complexe Polymérase d'un virus de la famille des *Paramyxoviridae.* La protéine P est un co-facteur de la polymérase (réplicase/transcriptase) virale et peut être phosphorylée. Les séquences de protéines P de *Paramyxoviridae* sont connues de l'homme du métier. Par exemple la protéine P du RSV humain souche Long présente une séquence de 241 acides aminés qui a été déposée dans la base de données Swissprot sous le numéro d'accès P12579. Cette séquence est montrée dans la séquence SEQ ID NO:7. La protéine P du RSV bovin comporte également 241 acides aminés (SEQ ID NO:8). Une protéine P du virus de Sendai (souche Harris), du virus de la rougeole (Souche Edmonston B), du virus de oreillons (souche SBL-1), et du *Metapneumovirus* humain (souche 00-1) sont également décrites dans la base de données Swissprot sous les numéros d'accès P04859 (SEQ ID NO:9), CAA91364 (SEQ ID NO:10), P19717 (SEQ ID NO:11), et Q91KZ5 (SEQ ID NO:12), respectivement. L'expression « protéine P » peut désigner une protéine P entière, une protéine P tronquée ou un fragment de la protéine P.

La protéine P des *Paramyxoviridae* forme des homo-oligomères, en particulier des homo-tétramères par exemple chez le virus de Sendai ou le RSV. Pour le RSV, un domaine de la protéine P capable de s'oligomériser (oligomérisation P-P) a été cartographié au niveau des acides aminés 120 à 150 de cette protéine (Castagné et al., 2004 ; Journal of General Virology ; 85 : 1643-1653). Ainsi, par exemple, le fragment constitué par les acides aminés 161 à 241 de la protéine P du RSV ne forme pas d'oligomères. Le domaine d'oligomérisation de la protéine P du virus de Sendai a été décrit par Tarbouriech et al. (2000; Nature Structural Biology ; 7, 777-781) comme étant constitué par les résidus 320 à 446 de la protéine P. D'autre part, la région d'oligomérisation P à été identifiée au niveau des acides aminés 304-376 pour la protéine P du virus de la rougeole (Johansson et al., 2003 ; Journal of Biological Chemistry ; 278, p 44567-44573).

Les séquences des protéines P et N décrites ci-dessus ont un caractère illustratif, ces séquences étant susceptibles de présenter des variations en fonction de la souche particulière considérée pour un virus donné. Ainsi, les positions d'acides aminés mentionnées dans la suite de la demande sont indiquées par rapport à ces séquences de référence. L'homme du métier est tout à fait à même d'identifier les domaines correspondants dans des souches de virus autres que celles exemplifiées, notamment à l'aide d'alignements de séquence réalisés par exemple avec des logiciels tels que Clustalw.

Les séquences codantes de ces protéines N et P de virus de la famille des *Paramyxoviridae* sont également connues de l'homme du métier.

Par « protéine étiquette », aussi appelée « protéine tag », on désigne une protéine qui est utilisée en fusion avec une protéine d'intérêt pour en faciliter la purification. Des protéines étiquettes sont connues de l'homme du métier. Des exemples de protéines étiquette incluent la glutathion-S-transférase (GST) ou les tag histidine qui sont des séquences comprenant généralement un enchaînement de 4 à 10 résidus histidine.

Une « protéine d'intérêt » désigne une protéine, un polypeptide ou un peptide (ces termes étant employés indifféremment l'un de l'autre) quelconque, comme par exemple une protéine marqueur, ou une protéine d'intérêt thérapeutique ou vaccinal.

Une protéine d'intérêt peut être par exemple la protéine GFP ("Green Fluorescent Protein") codée par le gène rapporteur gfp.

Une protéine d'intérêt thérapeutique peut par exemple être un polypeptide anti-angiogénique, par exemple RGD ou une séquence incluant RGD, l'endostatine, ou un polypeptide pro-apoptotique tel que l"'apoptosis inducing factor" (AIF), qui peuvent être utilisés comme agents anticancéreux, un polypeptide capable d'interagir spécifiquement avec des protéines virales et interférer avec les mécanismes permettant la réplication d'un virus, ou une toxine.

La protéine d'intérêt hétérologue peut être un antigène, en particulier une protéine antigénique d'intérêt vaccinal. Par « antigène » ou « Ag », on entend une séquence de nature peptidique ou glycopeptidique susceptible d'induire une réponse immunitaire chez un hôte auquel elle est administrée. Ainsi, un antigène peut être une protéine ou une partie de protéine (polypeptide) ou encore un petit peptide correspondant potentiellement à un épitope.

Un « épitope » est la partie d'un antigène qui est reconnue par un anticorps ou un récepteur lymphocytaire. Un épitope (linéaire) est généralement constitué d'une séquence de 7 à 15 acides aminés. Un antigène au sens de l'invention peut être constitué d'un épitope, comprendre un épitope, ou être une protéine antigénique.

Préférentiellement, l'antigène est un antigène dérivé d'un microorganisme pathogène, tel qu'un virus, une bactérie, un champignon, ou un organisme métazoaire ou protozoaire parasite.

Des exemples de virus incluent les virus de l'immunodéficience humaine (HIV), de l'hépatite B (HBV), de l'hépatite C (HCV), de l'herpès (*Herpes simplex),* de la grippe (virus influenza ou de la grippe aviaire), du Nil occidental, de la fièvre jaune, le cytomégalovirus, le papillomavirus (HPV), le virus d'Epstein-Barr (EBV), le RSV, le virus de la Dengue et le virus Chikungunya.

Des exemples de parasites incluent les parasites responsables de la malaria *(Plasmodium,* en particulier *P. falciparum, P. vivax, P. ovale* et *P. malariae),* et de trypanosomiase (en particulier, maladie du sommeil (*T. brucei*) et maladie de Chagas (*T*. *cruzi*))*.*

Le pathogène peut aussi être un champignon tel que *Candida albicans,* responsable de la candidose.

Des bactéries peuvent être par exemple *Helicobacter pylori, Clostridium tetani, Mycobacterium tuberculosis,* ou *Mycobacterium bovis.*

Par exemple un antigène peut être la GFP, une glycoprotéine virale telle que la protéine de fusion (F) du RSV, l'hémagglutinine du virus de la grippe, ou la protéine gp120 du HIV, une anatoxine bactérienne telle que l'anatoxine tétanique, ou un épitope de ceux-ci.

### Protéines de fusion protéine N-protéine d'intérêt et leur préparation

La demande décrit une protéine de fusion protéine N-protéine d'intérêt, comprenant une protéine N d'un virus de la famille des *Paramyxoviridae* et une protéine d'intérêt fusionnée en phase, en C-terminal de la protéine N.

Dans le contexte de la présente invention, la protéine d'intérêt est spécifiquement fusionnée en C-terminal de la protéine N. Cette construction permet à la protéine N d'être incorporée dans des structures en anneaux d'environ 10 nm de diamètre lorsqu'est employé un procédé de production de la protéine de fusion tel que décrit plus bas.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole, du *Metapneumovirus* humain, et du virus parainfluenza. De préférence, le virus est un *Pneumovirus,* en particulier le virus respiratoire syncytial (RSV), humain ou bovin.

La protéine N présente dans la protéine de fusion décrite ici a généralement la séquence d'acides aminés d'une protéine N native, c'est-à-dire naturellement présente chez un virus *Paramyxoviridae.*

Toutefois, pour les besoins de l'expression de la protéine de fusion, des modifications de séquence peuvent être apportées au niveau de l'extrémité C-terminale de la protéine N, sous réserve que la protéine N conserve sa capacité d'interagir avec la protéine P. En particulier, une protéine N native d'un virus *Paramyxoviridae* peut avoir été modifiée dans la région définie par les 25, de préférence les 20, 15, 10, ou 5, derniers acides aminés C-terminaux.

Ces modifications consistent typiquement en une suppression, substitution et/ou insertion d'un ou plusieurs acides aminés (par exemple de 1 à 25, ou 1 à 20, 1 à 15, 1 à 10, ou encore 1 à 5 acides aminés contigus ou non) dans la séquence C-terminale de la protéine N.

Un exemple de modification de séquence de la protéine N peut consister en une délétion des 6 ou 12 acides aminés C-terminaux, ces protéines N tronquées (NΔ6C et NΔ12C) étant encore capables d'interagir avec la protéine P.

La protéine d'intérêt peut être une protéine marqueur, une protéine d'intérêt thérapeutique ou d'intérêt vaccinal, sans s'y limiter. De préférence, la protéine d'intérêt est une protéine antigénique.

La protéine d'intérêt peut en outre être, elle-même, une protéine de fusion. Il peut s'agir par exemple d'une construction comprenant la GFP fusionnée en phase, à son extrémité C-terminale, avec une protéine d'intérêt, la GFP étant elle-même fusionnée en phase en C-terminal de la protéine N (soit une protéine de fusion protéine N-GFP-protéine d'intérêt).

Préférentiellement, la construction N-GFP-protéine d'intérêt peut inclure une séquence liaison entre les protéines N et GFP ayant pour séquence KLRILQSTVPSERPQASGVYMGNLTTRGPVAT (SEQ ID NO: 32) ce qui permet une optimisation du rendement de production de la protéine de fusion N-GFP (voir l'exemple 6) et donc de la protéine de fusion N-GFP-protéine d'intérêt.

La protéine d'intérêt peut être en particulier une protéine de fusion GFP-protéine antigénique ou GFP-protéine d'intérêt thérapeutique. Il peut également s'agir d'une protéine chimère comprenant une "séquence de liaison" fusionnée à la protéine d'intérêt. La séquence de liaison est un polypeptide qui comprend typiquement jusqu'à 30 acides aminés, de préférence jusqu'à 20 acides aminé, de préférence encore jusqu'à 10 acides aminés, et qui joue un rôle d'espaceur entre la protéine N et la protéine d'intérêt, ce qui permet à chacune de ces protéines d'être correctement repliée.

Ainsi, selon un mode de réalisation, la protéine d'intérêt est un antigène et la protéine de fusion selon l'invention est une fusion protéine N-antigène (« N-Ag »). De préférence, il s'agit d'une protéine de fusion de la protéine N du RSV humain ou bovin avec une protéine antigénique.

Comme décrit ici, la protéine d'intérêt peut être une protéine d'intérêt thérapeutique et la protéine de fusion décrite ici peut être une fusion protéine N-protéine d'intérêt thérapeutique. De préférence, il s'agit d'une protéine de fusion de la protéine N du RSV humain ou bovin avec une protéine d'intérêt thérapeutique.

Avantageusement, une étiquette (telle qu'un tag histidine) peut être fusionnée à l'extrémité N-terminale de la protéine N de manière à faciliter la purification des protéines de fusion protéine N-protéine d'intérêt.

Alternativement, la purification des protéines de fusion protéine N-protéine d'intérêt peut être effectuée en co-exprimant ladite protéine de fusion avec une protéine P du virus *Paramyxoviridae,* en particulier une protéine P fusionnée à la GST, comme il sera détaillé plus loin.

Pour produire les protéines de fusion selon l'invention, n'importe quelle technique conventionnelle de biologie moléculaire, de microbiologie ou d'ADN recombinant peut être employée. De telles techniques sont à la portée de l'homme, du métier et ont été décrites, notamment dans Sambrook, Fritsch & Maniatis, Molecular Cloning: A Laboratory Manual, Second Edition (1989) Cold Spring Harbor Laboratory Press, Cold Spring Harbor, New York ("Sambrook et al., 1989") ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et al. (eds.), Current Protocols in Molecular Biology, John Wiley & Sons, Inc. (1994).

La demande décrit donc également les acides nucléiques codant la protéine de fusion protéine N-protéine d'intérêt mentionnée ci-dessus

Une « séquence codante » désigne une séquence nucléotidique qui, lorsqu'elle est exprimée, résulte en la production d'un ARN, d'un polypeptide, d'une protéine, etc. Une séquence codante d'une protéine contient généralement un codon d'initiation (ATG) et un codon stop.

« Exprimer » ou « expression » signifie permettre ou faire en sorte que l'information contenue dans un gène ou une séquence d'ADN devienne manifeste, par exemple en produisant une protéine par activation des fonctions cellulaires impliquées dans la transcription et la traduction de la séquence génétique ou d'ADN correspondante. On parle de « coexpression » lorsque l'information contenue dans deux gènes ou séquences d'ADN est exprimée dans une même cellule hôte cellulaire.

La demande décrit donc des acides nucléiques (ADNc, ADN génomique, synthétique, ou ARN) codant les protéines de fusion protéine N-protéine d'intérêt. L'acide nucléique peut être double brin ou simple brin (c'est à dire un brin sens ou antisens). Les acides nucléiques ne sont pas limités à la séquence codant la protéine de fusion et peuvent inclure des séquences codantes ou non codantes en amont ou en aval de la séquence codant la protéine de fusion.

La séquence codant la protéine de fusion décrite ici est une séquence hybride ou chimère contenant au moins deux parties, consécutivement dans le sens 5'-3' une partie codant la protéine N et une partie du côté 3' codant la protéine d'intérêt. Lorsque la protéine d'intérêt est elle-même une fusion ou qu'une étiquette est attachée à la protéine N, la séquence codante contient le nombre approprié de parties. Entre chacune des parties, des codons peuvent coder une séquence de liaison.

La demande décrit aussi des vecteurs d'expression contenant les acides nucléiques codant la protéine de fusion protéine N-protéine d'intérêt. De tels vecteurs peuvent contenir un élément régulateur de la transcription lié de manière fonctionnelle avec l'ADN.

Une séquence codante est "liée de manière fonctionnelle avec" des séquences de contrôle transcriptionnel et traductionnel lorsque une ARN polymérase transcrit la séquence codante en ARN, en particulier en ARNm, qui peut ensuite être épissé s'il contient des introns, et traduit en la protéine codée par la séquence codante.

Les expressions "vecteur", "vecteur de clonage" et "vecteur d'expression" désignent le véhicule par lequel une séquence d'ADN ou d'ARN (par exemple un gène hétérologue) peut être introduite dans une cellule hôte de manière à transformer la cellule hôte et à promouvoir l'expression de la séquence introduite. Des exemples de vecteurs incluent les plasmides, les phages, les virus. Les vecteurs les plus communs sont les plasmides qui sont des unités de réplication autonomes, en général d'origine bactérienne, et qui peuvent être sous forme d'ADN double brin. Les plasmides peuvent intégrer facilement une séquence d'ADN exogène qui peut alors être facilement introduite dans un hôte approprié. Un vecteur plasmidique contient généralement une séquence d'ADN codante, une séquence d'ADN promotrice et présente un ou plusieurs sites de restriction permettant d'introduire un ADN exogène. Des exemples non limitatifs de plasmides incluent les plasmides pKK (Clonetech), pUC et pET (Novagen, Inc., Madison, WI), pRSET ou pREP (Invitrogen, San Diego, CA), pMAL (New England Biolabs, Beverly, MA), ou pGEX-4T-3 (Pharmacia).

Des cellules hôtes contenant les vecteurs d'expression sont également décrites. Ces cellules hôtes sont "transformées" avec lesdits vecteurs.

Par "cellule hôte" on entend n'importe quelle cellule qui est sélectionnée, modifiée, cultivée ou manipulée, pour la production d'une substance par la cellule, par exemple l'expression par la cellule d'un gène, d'une séquence d'ADN ou d'ARN, d'une protéine ou d'une enzyme.

Un "système d'expression" désigne une cellule hôte et un vecteur compatible utilisés dans des conditions appropriées pour produire une protéine codée par un ADN exogène porté par le vecteur et introduit dans la cellule hôte. Des systèmes d'expressions usuels incluent des cellules hôte *E. coli* et des vecteurs plasmidiques, des cellules d'insecte et des vecteurs Baculovirus ou encore des cellules de mammifère et des vecteurs avec promoteurs forts d'origine virale (par exemple cytomégalovirus).

Le système d'expression décrit ici est avantageusement un système d'expression bactérien, en particulier dans *E. coli,* avec par exemple comme vecteur pGEX-4T-3. Les systèmes bactériens sont en effet les systèmes d'expression qui permettent généralement d'obtenir les rendements de production les plus élevés.

La demande décrit donc également un procédé de production d'une protéine de fusion protéine N-protéine d'intérêt telle que définie plus haut qui comprend éventuellement la transformation d'une cellule hôte puis la culture de là cellule hôte transformée avec un vecteur comprenant un acide nucléique codant la protéine de fusion protéine N-protéine d'intérêt dans des conditions permettant l'expression de protéines de fusion protéine N-protéine d'intérêt, et éventuellement la purification des protéines de fusion protéine N-protéine d'intérêt exprimées. Les conditions de culture dépendent du système d'expression choisi (c'est-à-dire cellule hôte et vecteur) et la détermination de ces conditions est à la portée de l'homme du métier.

*Procédé de préparation de complexe protéine N-protéine d'intérêt*/*protéine P* Les Inventeurs ont précédemment montré que la protéine N d'un virus de la famille des *Paramyxoviridae* peut être produite dans un système de coexpression avec la protéine P du même virus de la famille des *Paramyxoviridae.* Ce même système peut être utilisé pour exprimer la protéine de fusion protéine N-protéine d'intérêt sous forme d'un complexe avec la protéine P, puis éventuellement purifier la protéine de fusion à partir de ce complexe.

La demande décrit un procédé de préparation d'un complexe protéine N-protéine d'intérêt/protéine P, les protéines N et P étant des protéines d'un virus de la famille des *Paramyxoviridae,* ledit procédé comprenant les étapes consistant à :
a) coexprimer une protéine de fusion protéine N d'un virus de la famille des *Paramyxoviridae-protéine* d'intérêt, dans laquelle la protéine d'intérêt est fusionnée en phase à l'extrémité C-terminale de la protéine N, avec une protéine P du même virus de la famille des *Paramyxoviridae ;*
b) recueillir les complexes protéine N-protéine d'intérêt/protéine P ainsi formés.

Comme spécifié plus haut, la protéine N peut porter, à son extrémité N-terminale, une étiquette telle qu'un tag histidine. En outre, la protéine d'intérêt peut elle-même être une construction fusion.

En outre, comme décrit plus haut, la protéine N peut être une protéine N native, ou, avoir été modifiée dans la région définie par les 25, de préférence les 20, 15, 10, ou 5, derniers acides aminés C-terminaux, sous réserve que la protéine N modifiée conserve la capacité d'interagir avec la protéine P. Ces modifications consistent typiquement en une suppression, substitution et/ou insertion d'un ou plusieurs acides aminés (par exemple de 1 à 25, ou 1 à 20, 1 à 15, 1 à 10, ou encore 1 à 5 acides aminés contigus ou non) dans la séquence C-terminale de la protéine N. Un exemple de modification de séquence de la protéine N peut consister en une délétion des 6 ou 12 acides aminés C-terminaux, ces protéines N tronquées (NΔ6C et NΔ12C) étant encore capables d'interagir avec la protéine P.

Préférentiellement, ladite protéine P est une protéine P tronquée ("PΔ") qui ne contient pas le domaine d'oligomérisation P et qui comprend un domaine de liaison à la protéine N. En effet, les Inventeurs ont précédemment montré que ce mode de réalisation permettait d'exprimer une grande quantité de complexes N-P sous forme soluble (demande de brevet FR 2885139). La solubilité est déterminée par centrifugation 30 minutes à 10 000 x g vis-à-vis d'un milieu aqueux, dépourvu de détergent, par exemple une solution saline telle que du PBS 1X (NaCl 140 mM, KCI 27 mM, Na₂HPO₄ 8 mM, KH₂PO₄ 1,5 mM, pH 7,4) ou un tampon Tris 10 mM pH 7,4, 150 mM NaCl.

L'expression "protéine P tronquée" désigne une protéine P dans laquelle une ou plusieurs séquences d'acides aminés contigus ont été supprimées. Il peut s'agir de la troncation d'une séquence C-terminale, d'une séquence N-terminale, d'une séquence « interne » par rapport à la structure primaire de la protéine P, ou d'une combinaison de ces troncatures.

L'invention concerne un procédé de préparation d'un complexe protéine N-protéine d'intérêt/protéine P, les protéines N et P étant des protéines d'un virus respiratoire syncytial (RSV), ledit procédé comprenant les étapes consistant à :
a) coexprimer une protéine de fusion protéine N d'un virus respiratoire syncytial - protéine d'intérêt, avec une protéine P tronquée du même virus respiratoire syncytial,
   dans laquelle la protéine d'intérêt est fusionnée en phase à l'extrémité C-terminale de la protéine N, la protéine N étant une protéine native ou une protéine N modifiée dans la région définie par les 25 derniers acides aminés C-terminaux, ladite protéine N conservant la capacité d'interagir avec la protéine P,
   et la protéine P tronquée ne contenant pas le domaine d'oligomérisation P et comprenant un domaine de liaison à la protéine N;
b) recueillir les complexes protéine N-protéine d'intérêt/protéine P ainsi formés.

Les protéines P tronquées selon l'invention ne contiennent pas le domaine d'oligomérisation P et sont capables d'interagir avec la protéine N, c'est-à-dire qu'elles présentent un domaine de liaison à la protéine N. Comme le domaine d'interaction de la protéine P des *Paramyxoviridae* avec la protéine N a été cartographié au niveau de l'extrémité C-terminale, des exemples de protéine P tronquée incluent préférentiellement un fragment C-terminal de la protéine P, ou une protéine P « chimère » constituée par la fusion d'un fragment C-terminal de la protéine P (capable d'interagir avec la protéine N) avec au moins une autre séquence d'acides aminés contigus de la protéine P. Ledit fragment C-terminal et ladite autre séquence de la protéine P ne sont pas eux-mêmes naturellement contigus, et ne présentent pas de recouvrement de séquence entre eux. Par exemple, une protéine P tronquée du RSV peut avoir la séquence constituée par les acides aminés 1 à 121 et 161 à 241 de la protéine P native. Un "fragment" d'un polypeptide de référence désigne n'importe quelle séquence d'acides aminés contigus trouvés dans la séquence du polypeptide de référence.

Par "fragment C-terminal de la protéine P" ou "PΔN" on entend une protéine P dans laquelle un ou plusieurs acides aminés consécutifs ont été supprimés à partir de l'extrémité N-terminale. De préférence, un fragment C-terminal de la protéine P désigne un enchaînement d'acides aminés positionnés dans la moitié C-terminale de la structure primaire de la protéine P (lorsque le nombre d'acides aminés de la séquence est impair, on peut attribuer arbitrairement un acides aminé supplémentaire à la moitié C-terminale de la protéine par rapport à la moitié N-terminale). Par exemple, pour la protéine P du RSV qui comprend 241 acides aminés, PΔ161N désigne un fragment C-terminal constitué par les acides aminés 161 à 241 de la protéine P. Par exemple également, pour la protéine P du virus de la rougeole (souche Edmonston B) qui comprend 507 acides aminés, PΔ386N désigne un fragment C-terminal constitué par les acides aminés 386 à 507 de la protéine P.

Lorsque la protéine P tronquée est un fragment C-terminal de la protéine P, il est alors décrit un procédé de préparation d'un complexe soluble protéine N-protéine d'intérêt/fragment C-terminal de la protéine P ("complexe N-PI/PΔN") d'un virus de la famille des *Paramyxoviridae,* ledit procédé comprenant les étapes consistant à :
a) coexprimer une protéine N d'un virus de la famille des *Paramyxoviridae,* fusionnée en phase à son extrémité C-terminale avec une protéine d'intérêt, avec un fragment C-terminal de la protéine P du même virus de la famille des *Paramyxoviridae,* ledit fragment C-terminal de la protéine P ne contenant pas le domaine d'oligomérisation P et étant capable d'interagir avec la protéine de fusion protéine N-protéine d'intérêt ;
b) recueillir les complexes solubles N-PI/PΔN ainsi formés.

Ledit virus de la famille *des Paramyxoviridae* peut être un *Paramyxovirinae* ou un *Pneumovirinae.* En particulier, le virus peut être sélectionné dans le groupe constitué du virus des oreillons, du virus de la rougeole, du *Metapneumovirus* humai, virus parainfluenza et du virus respiratoire syncytial. De préférence, le virus est un *Pneumovirus* tel que le virus respiratoire syncytial (RSV), humain ou bovin. Selon l'invention, le virus est un virus respiratoire syncytial (RSV), humain ou bovin.

L'homme du métier connaît ou est à même de déterminer des protéines P tronquées, ou plus spécifiquement des fragments C-terminaux de la protéine P, qui sont capables d'interagir avec la protéine de fusion antigène-protéine N.

Par exemple, la stratégie de co-expression des protéines N et P chez *E. coli* décrite par Castagné et al. (2004, Journal of General Virology ; 85 : 1643-1653) peut être utilisée pour cartographier le domaine d'interaction entre P et N. Les Inventeurs ont ainsi montré que des fragments C-terminaux de la protéine P du RSV, comprenant un oligopeptide constitué par les 9 acides aminés C-terminaux de la protéine P (acides aminés 233 à 241), sont capables d'interagir avec la protéine N.

Il a d'autre part été décrit, par exemple, que le domaine d'interaction de la protéine P du virus de Sendai, avec la protéine N sous forme de complexe ARN-N ou ribonucléoprotéine (RNP), appelé « X-domain » ou XD, est défini par les acides aminés 473 à 568 (Kolakofsky et al., 2004 ; Virology ; 318(2):463-73).

Les Inventeurs ont également mis en évidence que certains fragments C-terminaux de la protéine P du RSV, notamment le fragment PΔ161N (acides aminés 161 à 241), permettaient de préparer de grandes quantités de protéine N, comparativement à la protéine P entière qui, en pratique, ne permet pas d'obtenir des rendements suffisants à une échelle industrielle. Les mutants de délétion plus petits, jusqu'à PΔ233N (acides aminés 233 à 241) qui ne contient plus que 9 acides aminés, permettent d'obtenir des rendements comparables à ceux de PΔ161 N.

Ces fragments plus petits que PΔ161N correspondent à des fragments de la protéine du RSV capables d'interagir avec la protéine N et qui ne sont plus capables de s'oligomériser, donc ne contiennent plus le domaine d'oligomérisation P. Le domaine minimum d'oligomérisation P du RSV serait en effet défini par les acides aminés 120 à 150 de la protéine P.

Cette même stratégie a permis aux inventeurs de montrer qu'un fragment C-terminal de la protéine P du virus de la rougeole, constitué par les résidus d'acides aminés 386-507 (PΔ386N), interagissait avec la protéine N de ce virus et permettait sa purification. En revanche une délétion de la partie N-terminale de la protéine P, jusqu'à hauteur du résidu 456 (inclus ; fragment PΔ457N), ne permet pas de purifier la protéine N. La structure de la région C-terminale de la protéine P interagissant avec la ribonucléocapside a été déterminée par Johansson et al. (2003 Journal of Biological Chemistry vol. 278 p 44567-44573). La région d'oligomérisation P a été déterminée par délétion et prédiction comme étant définie par les acides aminés 304-376.

L'utilisation de fragments C-terminaux de la protéine P qui contiennent le domaine d'interaction avec la protéine N sous forme de RNP mais dans lesquels le domaine d'oligomérisation P a été délété permet donc à la fois l'interaction des fragments de la protéine P avec la protéine N, la formation de complexes solubles N-PΔN, et la production de ces complexes avec un rendement élevé. Sans vouloir se restreindre à un mécanisme particulier, il est supposé que l'absence du domaine d'oligomérisation P évite des problèmes d'insolubilité des complexes N-ΔPN liés à des interactions entre protéines P de ces complexes.

Ainsi, comme décrit ici, le procédé de préparation de complexe N-PI-PΔN implique l'expression d'un fragment C-terminal de la protéine P du RSV qui comprend les 9 derniers acides aminés C-terminaux de la protéine P du RSV et qui est dépourvu au moins des 119, de préférence, des 149, de préférence encore des 160 acides aminés N-terminaux de la protéine P du RSV.

Plus spécifiquement, dans le procédé décrit on peut coexprimer avec la protéine de fusion protéine N-protéine d'intérêt du RSV :
a) un fragment C-terminal de la protéine P du RSV qui comprend la séquence d'acides aminés 233 à 241 de la protéine P du RSV humain souche LONG telle que montrée dans SEQ ID NO:1, et qui s'étend en direction N-terminale jusqu'à un résidu d'acide aminé compris entre les positions 233 et 120, de préférence 150, de préférence encore 161, de la séquence de la protéine P du RSV telle que montrée dans SEQ ID NO:1, ou
b) un fragment C-terminal, homologue du fragment défini en a), d'une protéine P issue d'une autre souche du RSV humain ou d'une souche du RSV bovin.

Le fragment C-terminal de la protéine P du RSV peut par exemple être sélectionné dans le groupe constitué de PΔ120N (acides aminés 120 à 241 de P), PΔ150N (acides aminés 150 à 241 de P), PΔ161N (acides aminés 161 à 241 de P), PΔ180N (acides aminés 180 à 241 de P), PΔ200N (acides aminés 200 à 241 de P), PΔ220N (acides aminés 220 à 241 de P), PΔ230N (acides aminés 230 à 241 de P) et PΔ233N (acides aminés 233 à 241 de P).

La description concerne également un procédé dans lequel on coexprime, avec la protéine de fusion protéine N-protéine d'intérêt du RSV, une protéine P tronquée comprenant un fragment C-terminal de la protéine P du RSV tel que décrit ci-dessus, qui comprend les 9 derniers acides aminés C-terminaux de la protéine P du RSV et qui est dépourvu au moins des 119, de préférence des 149, de préférence encore les 160 acides aminés N-terminaux de la protéine P du RSV.

Par exemple, la protéine P tronquée comprenant un fragment C-terminal de la protéine P peut être constituée par la fusion des 122 derniers acides aminés N-terminaux avec les 80 derniers acides aminés C-terminaux de la protéine P du RSV ; elle peut par exemple être constituée par l'enchaînement des acides aminés 1 à 121 et 161 à 241 de la protéine P du RSV humain souche LONG telle que montrée dans SEQ ID NO:7.

Comme décrit ici, le *Paramyxoviridae* peut-être le virus de la rougeole et le procédé de préparation de complexe N-PI-PΔN peut impliquer l'expression d'un fragment C-terminal de la protéine P du virus de la rougeole comprenant au plus les 122 acides aminés C-terminaux de la protéine P ou étant constitué de ceux-ci. En particulier, il peut s'agir d'un fragment C-terminal constitué par les acides 386 à 507 de la protéine P (PΔ386N) du virus de la rougeole souche Edmonston B, telle que montrée dans SEQ ID NO:10, ou d'un fragment C-terminal, homologue à celui défini pour la protéine P de la souche Edmonston, d'une protéine P issue d'une autre souche de virus de la rougeole.

Dans le contexte de l'invention, le terme « homologue » a trait à la relation existant entre des protéines qui possèdent une même origine évolutive, par exemples des protéines homologues appartenant à différentes espèces, ou dans le cas de virus, de souches virales. De telles protéines (et leurs gènes codant), présentent des homologies de séquence, reflétées par leur similarité de séquences, que ce soit en terme de pourcentage de similitude ou en terme de présence de résidus ou motifs spécifiques au niveau de postions conservées.

L'expression «similarité de séquences » désigne le degré d'identité entre des séquences d'acides nucléiques ou d'acides aminés de protéines qui peuvent ou non partager une même origine évolutive. De manière usuelle, on utilise indistinctement les termes homologie ou similarité. Deux séquences d'acides aminés sont « essentiellement homologues » lorsque leurs acides aminés sont identiques à au moins 80%, ou similaires (c'est-à-dire fonctionnellement identiques) à au moins 90 %. Des séquences similaires ou homologues peuvent être identifiées par alignement, en utilisant par exemple les programmes BLAST ou FASTA.

Comme décrit ici, la protéine P, par exemple la protéine P tronquée et en particulier le fragment C-terminal de la protéine P, peut être exprimée sous forme de fusion avec une protéine facilitant la purification des complexes N-protéine d'intérêt/protéine P, notamment une protéine utilisable en chromatographie d'affinité. Il peut s'agir d'une protéine étiquette telle que la glutathion-S-transférase (GST), auquel cas la protéine de fusion protéine P-GST peut être isolée par chromatographie sur un support solide couplé au glutathion. D'autres étiquettes ou « Tag » peuvent être utilisés comme le poly-histidine ou « His-Tag ».

Avantageusement, selon le procédé de préparation de l'invention, la protéine P tronquée est exprimée sous forme de fusion avec une protéine facilitant la purification des complexes N-protéine d'intérêt/protéine P.

On obtient ainsi des complexes protéine N-protéine d'intérêt/protéine P-protéine étiquette (GST ou autre protéine étiquette en fusion avec la protéine P), dans lesquels la protéine étiquette peut être éliminée par clivage enzymatique. Par exemple, la GST peut être éliminée par clivage à la thrombine, ou par tout autre enzyme appropriée lorsque la fusion comprend une protéine autre que la GST.

Des exemples spécifiques de construction de vecteurs permettant de mettre en oeuvre le procédé selon l'invention sont décrits dans les exemples ci-dessous.

Selon un mode décrit ici, la protéine d'intérêt est un antigène et le procédé selon l'invention conduit à la formation de complexes solubles protéine N-antigène/protéine P (complexe N-Ag/P), de préférence protéine N-antigène/protéine P tronquée (complexe N-Ag/PΔ) et de préférence encore protéine N-antigène/fragment C-terminal de la protéine P (N-Ag/PΔN).

### Séparation des protéines de fusion à partir des complexes protéine N-protéine d'intérêt/protéine P

Le procédé de préparation d'un complexe protéine N-protéine d'intérêt/protéine P, la protéine P pouvant être tronquée et être en particulier un fragment C-terminal de la protéine P, tel que décrit ci-dessus permet aisément d'obtenir des complexes protéine N-protéine d'intérêt/protéine P sous forme isolée ou purifiée.

La demande décrit également un complexe protéine N-protéine d'intérêt/protéine P, les protéines N et P étant des protéines d'un virus de la famille des *Paramyxoviridae,* susceptibles d'être obtenus par un procédé de préparation tel que décrit ci-dessus.

L'invention concerne un complexe protéine N-protéine d'intérêt/protéine P sous forme d'anneaux solubles comprenant la protéine de fusion protéine N-protéine d'intérêt avec leur ARN, les protéines N et P étant des protéines d'un virus respiratoire syncytial, dans lequel la protéine d'intérêt est fusionnée en phase à l'extrémité C-terminale de la protéine N, la protéine N étant une protéine native ou une protéine N modifiée dans la région définie par les 25 derniers acides aminés C-terminaux, ladite protéine N conservant la capacité d'interagir avec la protéine P, la protéine P étant une protéine P tronquée du même virus respiratoire syncytial ne contenant pas le domaine d'oligomérisation P et comprenant un domaine de liaison à la protéine N.
Dans un mode particulier, la protéine d'intérêt du complexe selon l'invention est une protéine marqueur, ou une protéine d'intérêt vaccinal. La protéine d'intérêt vaccinal pouvant être un antigène dérivé d'un microorganisme pathogène. Dans une variante, la protéine d'intérêt est une construction comprenant la GFP (Green Fluorescent Protein) fusionnée en phase, à son extrémité C-terminale, avec une protéine vaccinale.

En particulier, l'invention concerne un complexe protéine N-protéine d'intérêt/protéine P tel que décrit ci-dessus, dans lequel la protéine de fusion protéine N-protéine d'intérêt est une protéine de fusion N-GFP-protéine d'intérêt comprenant la GFP (Green Fluorescent Protein) fusionnée en phase à l'extrémité C-terminale de la protéine N, incluant une séquence liaison entre les protéines N et GFP ayant pour séquence SEQ ID NO: 32.

Il est décrit en particulier un complexe soluble protéine N-protéine d'intérêt/protéine P tronquée (complexe N-PI/PΔ), et plus spécifiquement un complexe soluble protéine N-protéine d'intérêt/fragment C-terminal de la protéine P (complexe N-PI/PΔN), N et P étant des protéines d'un virus de la famille des *Paramyxoviridae.*

A partir de ces complexes protéine N-protéine d'intérêt/protéine P, ou plus spécifiquement N-PI/PΔ ou encore N-PI/PΔN, la protéine de fusion protéine N-protéine d'intérêt peut être aisément isolée sous forme d'anneaux, avec leur ARN, par exemple par chromatographie d'exclusion de taille (gel filtration). Cette séparation peut être mise en oeuvre, le cas échéant, après séparation, par clivage enzymatique, de la protéine P et de la protéine étiquette à laquelle la protéine P est éventuellement fusionnée.

Il est décrit donc en outre un procédé de préparation de protéines de fusion protéine N d'un virus de la famille des *Paramyxoviridae-protéine* d'intérêt, ledit procédé comprenant les étapes consistant à :
a) préparer un complexe protéine N-protéine d'intérêt/protéine P par un procédé tel que défini plus haut ; et
b) séparer les protéines de fusion protéine N-protéine d'intérêt des complexes protéine N-protéine d'intérêt/protéine P.

L'invention concerne donc en outre un procédé de préparation d'une protéine N d'un virus respiratoire syncytial, fusionnée en phase à son extrémité C-terminale avec une protéine d'intérêt, ledit procédé comprenant la séparation de la protéine de fusion protéine N-protéine d'intérêt à partir du complexe protéine N-protéine d'intérêt/protéine P selon l'invention.

La protéine P est une protéine tronquée, de préférence est un fragment C-terminal de la protéine P.

Il est plus particulièrement décrit un procédé de préparation de protéines de fusion solubles protéine N-protéine d'intérêt d'un virus de la famille des *Paramyxoviridae,* ledit procédé comprenant les étapes consistant à :
a) préparer un complexe soluble protéine N-protéine d'intérêt/fragment C-terminal de la protéine P (complexe N-PI/PΔN) par un procédé tel que défini plus haut ; et
b) séparer les protéines de fusion protéine N-protéine d'intérêt des complexes solubles N-PI/PΔN.

Comme décrit plus haut, la protéine N peut être une protéine N native, ou, avoir été modifiée dans la région définie par les 25, de préférence les 20, 15, 10, ou 5, derniers acides aminés C-terminaux, sous réserve que la protéine N modifiée conserve la capacité d'interagir avec la protéine P. Ces modifications consistent typiquement en une suppression, substitution et/ou insertion d'un ou plusieurs acides aminés (par exemple de 1 à 25, ou 1 à 20, 1 à 15, 1 à 10, ou encore 1 à 5 acides aminés contigus ou non) dans la séquence C-terminale de la protéine N. Un exemple de modification de séquence de la protéine N peut consister en une délétion des 6 ou 12 acides aminés C-terminaux, ces protéines N tronquées (NΔ6C et NΔ12C) étant encore capables d'interagir avec la protéine P.

Sont également décrites les protéines de fusion protéine N-protéine d'intérêt susceptibles d'être obtenues par le procédé ci-dessus.

L'invention concerne une protéine de fusion protéine N-protéine d'intérêt, isolée, structurée en anneaux solubles comprenant la protéine de fusion protéine N-protéine d'intérêt avec leur ARN, susceptible d'être obtenue par le procédé de préparation d'une protéine N d'un virus respiratoire syncytial selon l'invention, où la protéine N est une protéine N d'un virus respiratoire syncytial native ou modifiée dans la région définie par les 25 derniers acides aminés C-terminaux, et la protéine d'intérêt est fusionnée en phase, en C-terminal de la protéine N.

La protéine d'intérêt peut être une protéine d'intérêt thérapeutique ou vaccinal. Préférentiellement, la protéine d'intérêt est un antigène.

### Compositions immunogènes, vaccinales ou thérapeutiques

Les Inventeurs ont précédemment montré que la protéine N du RSV structurée en anneau, isolée ou sous forme de complexe protéine N-fragment C-terminal de la protéine P (N-PΔN), est fortement immunogène, et qu'elle permet notamment de stimuler une réponse locale par exemple au niveau de la muqueuse respiratoire.

Les Inventeurs ont désormais mis en évidence que ces structures en anneaux peuvent être utilisées en tant que vecteur de protéines thérapeutiques ou vaccinales.

L'utilisation d'une protéine N d'un virus de la famille des *Paramyxoviridae* comme vecteur de molécules ou protéines d'intérêt thérapeutique ou vaccinale est décrite.

Comme décrit ici, une protéine thérapeutique ou vaccinale est fusionnée à la protéine N, comme décrit plus haut.

Comme décrit ici, une molécule thérapeutique ou vaccinale peut-être couplée chimiquement à la protéine N. En effet, en ajoutant une cystéine en C-terminal de la protéine N, on crée un site permettant de coupler chimiquement diverses molécules de nature protéique ou non protéique (organique ou minérale). On peut greffer une protéine portant également une cystéine par pont disulfure. Le maléimide couplé lui-même à diverses molécules peut ainsi être greffé sur cette cystéine par une liaison covalente. Le maléimide peut être couplé à toutes sortes de molécules organiques, telles que le dextron, la biotine, l'or, ou toute protéine.

Il est donc décrit aussi un conjugué de la protéine N d'un virus de la famille des *Paramyxoviridae* avec une molécule d'intérêt, en particulier une molécule thérapeutique ou vaccinale.

Est également décrite une méthode d'administration d'une molécule ou protéine thérapeutique ou vaccinale à un sujet, comprenant l'administration de ladite molécule thérapeutique ou vaccinale liée de manière covalente à une protéine N d'un virus de la famille des *Paramyxoviridae* audit sujet, ou comprenant l'administration de ladite protéine sous forme d'une protéine de fusion avec la protéine N d'un virus de la famille des *Paramyxoviridae,* la protéine thérapeutique ou vaccinale étant fusionnée en phase, en C-terminal de la protéine N.

Est également décrite une composition pharmaceutique, comprenant une protéine de fusion protéine N-protéine d'intérêt, éventuellement sous forme de complexe protéine N-protéine d'intérêt/protéine P, les protéines N et P étant d'un même virus de la famille des *Paramyxoviridae,* dans un véhicule pharmaceutiquement acceptable.

L'invention propose une composition pharmaceutique, comprenant une protéine N d'un virus respiratoire syncytial fusionnée avec une protéine d'intérêt selon l'invention ou un complexe protéine N-protéine d'intérêt/protéine P selon l'invention, et un véhicule pharmaceutiquement acceptable.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole, et du virus parainfluenza. De préférence, le virus est un *Pneumovirus,* en particulier le virus respiratoire syncytial (RSV), par exemple humain ou bovin.

La protéine d'intérêt peut être une protéine d'intérêt thérapeutique ou vaccinal.

Comme décrit ici, la composition pharmaceutique est adaptée à un usage thérapeutique. Selon cet exemple hors invention, la protéine d'intérêt est une protéine d'intérêt thérapeutique. Les Inventeurs ont en effet déterminé que la protéine de fusion protéine N-protéine d'intérêt pénètre dans les cellules et que la protéine N constitue donc à ce titre un vecteur potentiel pour des molécules ou protéines d'intérêt thérapeutique, comme par exemple un polypeptide anti-angiogénique ou pro-apoptotique.

Selon un mode de réalisation, la composition pharmaceutique est adaptée à un usage immunogène ou vaccinal. Selon ce mode de réalisation, la protéine d'intérêt est une protéine antigénique, de préférence un antigène dérivé d'un microorganisme pathogène, tel qu'un virus, une bactérie, un champignon, ou un organisme métazoaire ou protozoaire parasite.

Les Inventeurs ont en effet montré que la réponse d'un hôte vis-à-vis d'un antigène est très significativement augmentée lorsque cet antigène est présenté sous forme de protéine de fusion avec la protéine N. Les protéines de fusion protéine N-antigène sont capables de stimuler la réponse immunitaire par voie cellulaire comme humorale.

Les protéines de fusion solubles protéine N-antigène peuvent être utilisées en vaccination sous forme de complexe avec la protéine P sans effet défavorable. En conséquence, une composition immunogène ou vaccinale selon l'invention peut comprendre un complexe soluble protéine N-antigène/protéine P, en association avec un véhicule pharmaceutiquement acceptable.

Par "véhicule pharmaceutiquement acceptable", on entend tout solvant, milieu de dispersion, agents retardant l'absorption etc, qui ne produit pas de réaction secondaire, par exemple allergique, chez l'humain ou l'animal.

Des exemples de véhicules physiologiquement acceptables sont connus de l'homme du métier. Des exemples de véhicules liquides incluent des solutions aqueuses stériles qui ne contiennent aucun matériau autre que les actifs et de l'eau, ou qui contiennent un tampon tel que du phosphate de sodium à une valeur de pH physiologique, à une salinité physiologique, ou aux deux tel qu'une solution saline tamponnée au phosphate (PBS). Les véhicules aqueux peuvent contenir plus d'un sel tampon, de même que des sels tels que du chlorure de sodium ou potassium, du dextrose, du polyéthylène glycol et d'autres solutés.

Les compositions sont administrées de manière compatible avec la formulation galénique et en quantité thérapeutiquement efficace. La quantité à administrer dépend du sujet à traiter, de la capacité du système du sujet à utiliser le principe actif, et le degré d'effet thérapeutique désiré. Les quantités précises de protéine de fusion que nécessite l'administration dépendent du choix du praticien et des particularités de chaque individu.

Avantageusement, lorsque la composition pharmaceutique est une composition immunogène ou vaccinale, elle peut en outre comprendre un adjuvant. Un "adjuvant" désigne un produit qui augmente, stimule, active, renforce, ou module la réaction immunitaire au niveau cellulaire ou humoral dirigée contre un antigène administré simultanément. Des exemples d'adjuvants classiques incluent des adjuvants contenant des antigènes bactériens, tels que l'adjuvant complet de Freund, le LPS et ses dérivés, les toxines bactériennes (toxine cholérique et entérotoxine) et leurs mutants détoxifiés (par exemple LT(R192G)), des séquences oligonucléotidiques contenant des motifs CpG, des adjuvants minéraux tels que l'hydroxyde d'aluminium (Alum), le phosphate de calcium ou le phosphate de potassium, des émulsions huileuses et agents émulsifiants (saponines, par exemple QS21), des cytokines.

Les compositions immunogènes selon l'invention permettent d'induire une réponse immunitaire contre l'antigène chez le sujet vacciné, ou plus spécifiquement contre le pathogène dont dérive l'antigène.

Les compositions vaccinales selon l'invention permettent de conférer une protection vis-à-vis d'une infection par un pathogène comportant l'antigène, c'est-à-dire une réduction de la gravité des effets d'une telle infection par rapport à un sujet non immunisé avec la composition vaccinale.

Est également décrite l'utilisation d'une composition vaccinale telle que définie plus haut dans une méthode de vaccination contre le pathogène dont dérive l'antigène.

La demande décrit également une méthode de vaccination comprenant au moins une administration d'une composition vaccinale à un sujet. De préférence, la méthode de vaccination comprend une première administration à un sujet d'une composition vaccinale, et au moins une administration de rappel de ladite composition vaccinale au même sujet. Les administrations de rappel, en réexposant le patient à l'antigène, induisent une réponse immunitaire secondaire plus forte.

La composition vaccinale est avantageusement administrée en une quantité efficace pour induire une réponse immunitaire protectrice ou thérapeutique vis-à-vis d'une infection par le pathogène dont dérive l'antigène. La posologie dépend naturellement de l'actif considéré, du mode d'administration, de l'âge du sujet et de son état. La quantité de complexe N-Ag-P, N-Ag-PΔ, ou N-Ag-PΔN, ou de protéine de fusion N-Ag par dose peut être comprise entre 0,1 et 200 μg, et préférablement comprise entre 10 et 100 μg par dose vaccinale.

La composition pharmaceutique, immunogène ou vaccinale peut être administrée par n'importe quelle voie, en particulier par voie mucosale (par exemple oculaire, intranasale, orale), ou par voie parentérale (par exemple sous-cutanée, intradermique, intramusculaire, intraveineuse, ou intrapéritonéale).

L'expression "sujet" désigne un humain ou un animal non-humain, par exemple un oiseau ou un mammifère tel qu'un bovin, un ovin, un rongeur, un canidé en particulier un chien, un félin en particulier un chat, un porc, un singe, exposé ou susceptible d'être exposé à une infection par un virus *Paramyxoviridae* ou par toute autre pathologie. Préférentiellement un sujet au sens de l'invention est un humain ou un bovin.

Pour chacun de ces aspects, et comme décrit plus haut, la protéine N peut être une protéine N native, ou, avoir été modifiée dans la région définie par les 25, de préférence les 20, 15, 10, ou 5, derniers acides aminés C-terminaux, sous réserve que la protéine N modifiée conserve la capacité d'interagir avec la protéine P. Ces modifications consistent typiquement en une suppression, substitution et/ou insertion d'un ou plusieurs acides aminés (par exemple de 1 à 25, ou 1 à 20, 1 à 15, 1 à 10, ou encore 1 à 5 acides aminés contigus ou non) dans la séquence C-terminale de la protéine N. Un exemple de modification de séquence de la protéine N peut consister en une délétion des 6 ou 12 acides aminés C-terminaux, ces protéines N tronquées (NΔ6C et NΔ12C) étant encore capables d'interagir avec la protéine P.

### Applications diagnostiques

La fusion d'un antigène comprenant au moins un épitope avec la protéine N d'un virus des *Paramyxoviridae* constitue par ailleurs un réactif susceptible d'être utilisé dans des applications diagnostiques pour la détection d'anticorps dirigés contre au moins un desdits épitopes portés par la protéine de fusion.

Il est décrit un réactif diagnostic comprenant une protéine de fusion protéine N-antigène comprenant un antigène fusionné en phase en C-terminal d'une protéine N d'un virus de la famille des *Paramyxoviridae,* telle que décrite plus haut.

L'invention concerne donc en outre un réactif diagnostic comprenant une protéine de fusion selon l'invention, dans laquelle la protéine d'intérêt est un antigène.

Un kit diagnostic comprenant ledit réactif et des moyens de détection appropriés est également décrit.

Ledit antigène comprend au moins un épitope, et peut également comprendre deux ou plus de deux épitopes identiques ou différents.

L'utilisation d'une protéine de fusion protéine N-antigène pour la détection, *in vitro* ou *in vivo,* d'anticorps dirigés contre ledit antigène de la protéine de fusion est également décrite.

L'invention propose également l'utilisation d'une protéine de fusion selon l'invention comprenant un antigène fusionné en phase en C-terminal d'une protéine N d'un virus respiratoire syncytial pour la détection *in vitro* d'anticorps dirigés contre ledit antigène de la protéine de fusion.

Lorsque l'antigène comprend un unique épitope, les anticorps détectés sont alors spécifiques de cet épitope.

Lorsque l'antigène comprend deux ou plus de deux épitopes différents, les anticorps détectés peuvent être spécifiques d'un, deux ou plus de deux des épitopes dudit antigène.

Une méthode de détection d'anticorps spécifiques d'un antigène, dans un échantillon biologique est décrite, comprenant les étapes consistant à :
a) mettre en contact ledit échantillon biologique avec une protéine de fusion N-antigène comprenant un antigène fusionné en phase en C-terminal d'une protéine N d'un virus de la famille des *Paramyxoviridae,*
b) détecter les complexes protéine de fusion N-antigène/anticorps formés,

la présence de tels complexes étant révélatrice de la présence d'anticorps spécifiques de l'antigène dans l'échantillon biologique.

L'invention concerne une méthode de détection d'anticorps spécifiques d'un antigène, dans un échantillon biologique, comprenant les étapes consistant à :
a) mettre en contact ledit échantillon biologique avec une protéine de fusion selon l'invention comprenant un antigène fusionné en phase en C-terminal d'une protéine N d'un virus respiratoire syncytial,
b) détecter les complexes protéine de fusion N-antigène/anticorps formés, la présence de tels complexes étant révélatrice de la présence d'anticorps spécifiques de l'antigène dans l'échantillon biologique.

L'échantillon biologique peut être un échantillon de tissu obtenu par exemple par biopsie musculaire, hépatique, cardiaque, cérébrale, etc, ou un échantillon liquide, par exemple un liquide biologique tel que le sang, le plasma, ou le liquide céphalorachidien.

La détection des complexes peut être réalisée par des moyens usuels bien connus de l'homme du métier tels que la chromatographie (d'exclusion de taille, d'affinité, etc) ou l'électrophorèse en conditions non dénaturantes.

La détection des complexes N-antigène/anticorps peut par ailleurs être facilitée en marquant les protéines N de manière détectable.

Pour chacun de ces aspects, la protéine N peut être une protéine N native, ou, avoir été modifiée dans la région définie par les 25, de préférence les 20, 15, 10, ou 5, derniers acides aminés C-terminaux, sous réserve que la protéine N modifiée conserve la capacité d'interagir avec la protéine P. Ces modifications consistent typiquement en une suppression, substitution et/ou insertion d'un ou plusieurs acides aminés (par exemple de 1 à 25, ou 1 à 20, 1 à 15, 1 à 10, ou encore 1 à 5 acides aminés contigus ou non) dans la séquence C-terminale de la protéine N. Un exemple de modification de séquence de la protéine N peut consister en une délétion des 6 ou 12 acides aminés C-terminaux, ces protéines N tronquées (NΔ6C et NΔ12C) étant encore capables d'interagir avec la protéine P.

Les exemples et figures suivantes illustrent l'invention sans en limiter sa portée.

### FIGURES

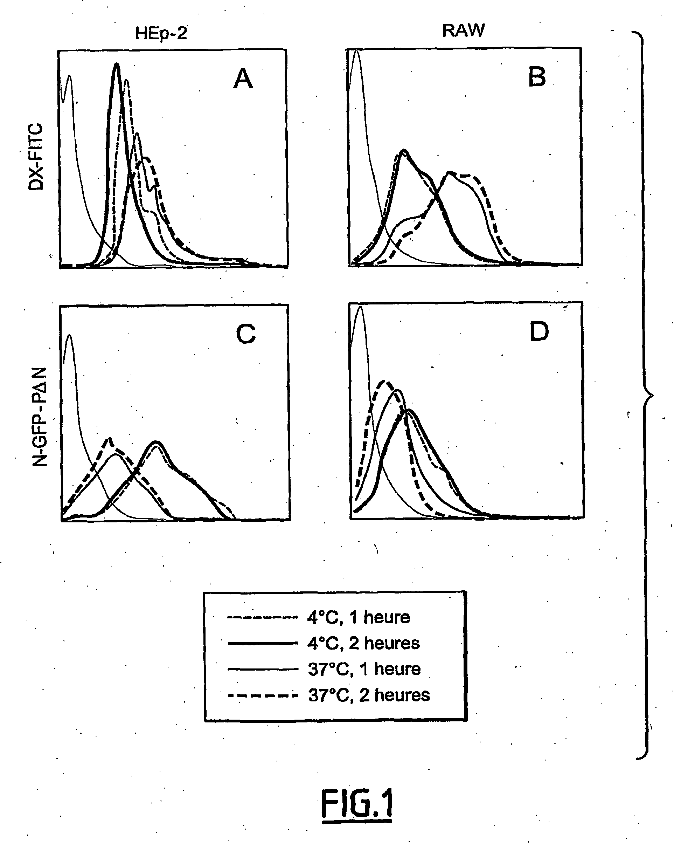

La figure 1 montre la capture des complexes N-GFP-PΔN par des cellules HEp2 ou RAW. Les cellules HEp-2 (A ; C) ou RAW (B ; D) sont incubées en présence de DX-FITC (A ; B) ou N-GFP-PΔN(C ; D). L'incubation dure une heure à 4°C (courbes en trait pointillé) ou deux heures à 4°C (courbes en trait continu épais), ou une heure à 37°C (courbes en trait continu) ou deux heures à 37 °C (courbes en trait pointillé épais). La fluorescence (FITC ou GFP) est analysée en cytométrie en flux sur 100 000 évènements.

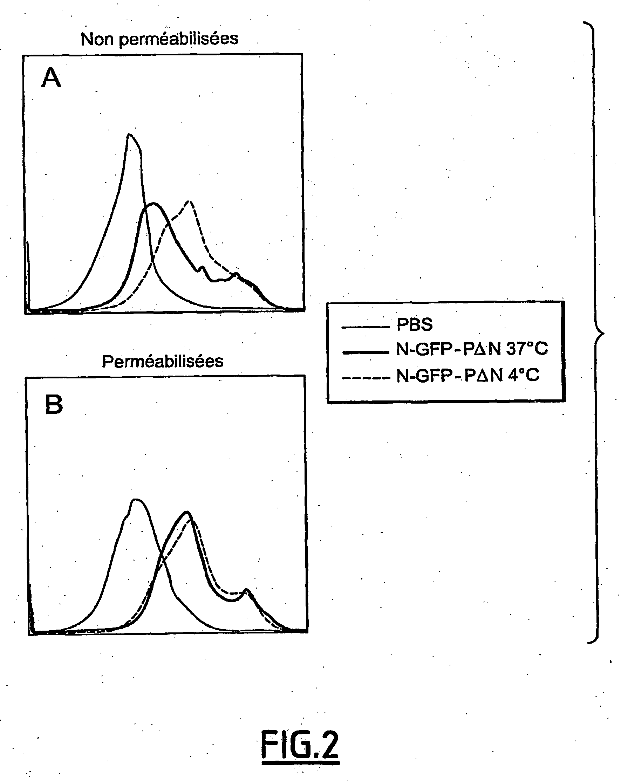

La figure 2 montre que les complexes N-GFP-PΔN sont internalisées dans les cellules RAW. Les cellules RAW sont incubées pendant une heure en présence de N-GFP-PΔN à 4°C (courbes en trait continu épais) ou à 37°C (courbes en trait pointillé). La protéine N est détectée par immunomarquage indirect en cytométrie en flux sur des cellules non perméabilisées (A) ou des cellules perméabilisées (B). L'autofluorescence des cellules est donnée par le signal des cellules incubées à 37°C en PBS (courbes en trait continu).

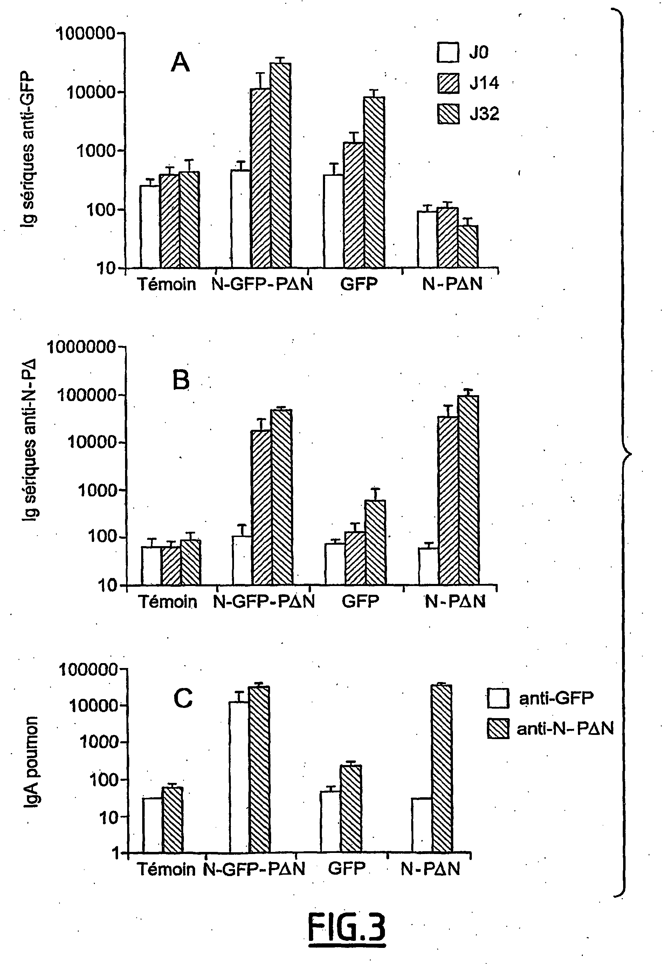

La figure 3 montre l'augmentation de la réponse anticorps par la vectorisation de la GFP sur les complexes N-PΔN. Des souris BALB/c sont immunisées à J0 par voie nasale, avec l'adjuvant seul (témoin), le complexe N-GFP-PΔN, N-PΔN ou la GFP, toujours en présence d'adjuvant. Un rappel est réalisé après deux semaines (J14). Les animaux sont euthanasiés deux semaines après le rappel (J28). Le sérum est collecté à J0, J14 et J28 (A et B). Les lavages broncho-alvéolaires sont réalisés à J28 (C). Le titre en anticorps anti N-PΔN (B et C) et anti-GFP (A et C) est déterminée par ELISA. Les données sont exprimées comme la moyenne ± erreur standard à la moyenne (n=8 pour les groupes témoin et GFP, n=6 pour le groupe N-GFP-PΔ, n=4 pour le groupe N-PΔ) et représentées selon une échelle logarithmique.

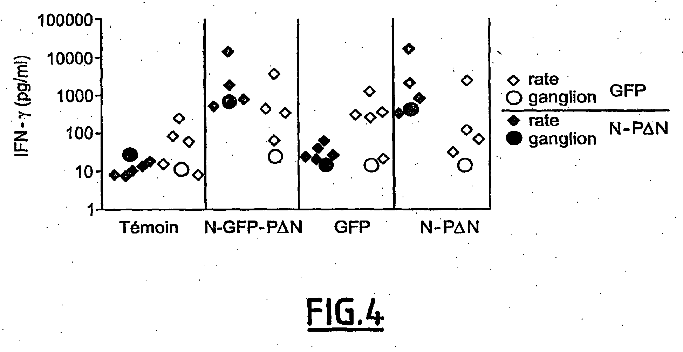

La figure 4 illustre l'absence de réponse T mémoire contre la GFP, y compris sous sa forme vectorisée par N-PΔN. Des souris BALB/c sont immunisées à J0 par voie nasale avec l'adjuvant seul (témoin), les complexes N-GFP-PΔN, N-PΔN ou la GFP, toujours en présence d'adjuvant. Un rappel est réalisé après deux semaines (J14). Les animaux sont autopsiés deux semaines après le rappel (J28) pour prélever les rates et les ganglions loco-régionaux. Les suspensions cellulaires sont traitées individuellement pour les rates (losanges) et en pool par groupe pour les ganglions (ronds). Les cellules sont restimulées pendant 72h avec GFP (losanges et ronds blancs) ou N-PΔN (losanges et ronds noirs). La sécrétion d'IFN-γ est mesurée par ELISA. Quand les cellules sont cultivées dans du milieu seul, le niveau basal d'IFN-γ est inférieur à 15 pg/ml.

### EXEMPLES

### Exemple 1 : Construction des plasmides contenant la région C-terminale de la phosphoprotéine du RSV et la protéine N du RSV fusionnée à la GFP.

La protéine P du RSV souche Long est composée de 241 résidus d'acides aminés.

Séquences des amorces oligonucléotidiques (de 5' vers 3') utilisées pour amplifier la partie C-terminale de la protéine P du RSV (les sites de restriction *Bam*HI sont soulignés ; le codon de démarrage (« start ») ATG du gène P est figuré en caractères gras) :
LONG-PBam+ : GAGGGATCCATC**ATG**GAAAAGTTTGCTCCTG (SEQ ID NO:13)
LONG-P- : CTGTTGGTGTTGTGTGTTGAAGTGCAG (SEQ ID NO:14)
P161B+ : GAGGGATCCTCTGCTAGGGATGGTATAAGAG (SEQ ID NO:15)
P180B+ : GAGGGATCCAAAATCAGAACTGAAGCATTAATGACC (SEQ ID NO:16)
P201 B+ : GAGGGATCCGAGGAAAGTGAAAAGATGGCAAAAG (SEQ ID NO:17)
P221 B+ : GAGGGATCCGAGAAATTGAACAACCTGTTGG (SEQ ID NO:18)
P230NB+ : GATCCAATGATAGTGACAATGATCTATCACTTGAAGATTTCTGA (SEQ ID NO:19)
P230N- : TCAGAAATCTTCAAGTGATAGATCATTGTCACTATCATTG (SEQ ID NO:20)

L'ADNc du gène P du RSV souche Long a été amplifié par RT-PCR à partir de cellules Hep-2 infectées par la souche Long du RSV humain en utilisant les amorces LONG-PBam+ et LONG-P- (Castagné et al., 2004 ; Journal of General Virology ; 85 : 1643-1653). Le produit de PCR a été digéré par l'enzyme de restriction *Bam*HI et cloné dans le plasmide pGEX-4T-3 (Pharmacia) aux sites *Bam*HI-*Sma*I en phase avec le gène codant pour la Glutathion-S-Transferase ou GST. Le plasmide s'appelle pGEX-P.

### • Clonage de P161-241 (PΔ161N)

La région C-terminale de P (acides aminés 161-241) a été amplifiée par PCR à partir du plasmide pGEX-P dans les conditions suivantes :
Amorces de PCR : P161 B+ et LONG-P-100 ng chaque (1μl chaque)
ADN matrice pGEX-P : 10 ng (1μl)
Enzyme : Pfu Turbo marque Stratagene (2,5 U par μl) : 1 μl
dATP : 0.2 mM final
dGTP : 0.2 mM final
dCTP : 0.2 mM final
dTTP : 0.2 mM final
Tampon Pfu 1 X final (Stratagene)
Volume final : 100 μl

La PCR a été réalisée dans les conditions suivantes :
5 cycles : 15 secondes à 94°C, 2 minutes à 40°C, 1 minute à 72°C ;
25 cycles : 15 secondes à 94°C, 1 minutes à 55°C, 1 minute à 72°C

L'ADN amplifié a été extrait par un volume (100 μl) de phénol/chloroforme (1vol/1vol), puis par un volume de chloroforme, enfin précipité par ajout d'un dixième de volume de NaCl 5M (10 µl) et deux volumes d'éthanol à 100% (200 µl). L'ADN a été centrifugé 20 minutes à 13000 g, lavé avec un volume d'éthanol à 70%, séché, resuspendu dans un volume d'eau de 90 µl. Après ajout de 10 µl de tampon 10X pour l'enzyme *Bam*HI, l'ADN a été digéré 2 heures à 37°C en présence de 10 unités d'enzyme *Bam*HI. L'ADN digéré a été déposé sur un gel d'agarose à 1,5 % dans du tampon Tris-Borate-EDTA (TBE) 1X en présence de bromure d'éthidium et mis à migrer par électrophorèse. La bande correspondant à l'ADN de P161-241 a été découpée et l'ADN extrait par électroélution. Il a été à nouveau extrait avec un volume de phénol-chloroforme, un volume de chloroforme et précipité à l'éthanol. Il a été ligaturé avec le vecteur pGEX-4T-3 digéré par *Bam*HI et *Sma*I après purification en gel d'agarose à 1% :
ADN pGEX4T-3 : 100ng
ADN P161-241 : 100 ng
Tampon ligase 1X final
Ligase (5U/µl) : 1 µl
Volume final 20 µl

L'ensemble a été incubé une nuit à 14°C. Le lendemain des bactéries DH5-alpha TM (Life Technologies) compétentes ont été transformées avec 10 µl de produit de ligation et étalées sur boîte de Pétri contenant du milieu LB-agar supplémenté avec 100 µg/ml d'ampicilline final. Les colonies de bactéries recombinantes ont été criblées par mini préparation de plasmide et digestion par les enzymes de restriction *Bam*HI et *Xho*I*.* Les plasmides recombinants montrent alors deux bandes en gel d'agarose, une correspondant au vecteur (4,9 kb) et la seconde correspondant à la partie C-terminale de P (246 pb). Les plasmides recombinants ont été entièrement séquencés.

### • Clonage de P180-241, P201-241, P221-241

Les fragments de P correspondants aux parties d'acides aminés 180-241, 200-241, 220-241 ont été obtenus par PCR à partir du plasmide pGEX-P en utilisant les amorces suivantes :
P180-241 : amorces P180B+ et LONG-P
P200-241 : amorces P201 B+ et LONG-P-
P220-241 : amorces P221 B+ et LONG-P-
Ils ont été amplifiés et clonés de la même façon que P161-241 (voir au-dessus).

### • Clonage du gène codant pour la protéine de nucléocapside du RSV souche Long.

Le gène codant pour la protéine N du RSV humain souche Long a été obtenu par RT-PCR à partir de cellules Hep-2 infectées par ce virus. Les amorces utilisées étaient :
LONG-Nbam+ : GAGGGATCCATGGCTCTTAGCAAAGTCAAGTTG (SEQ ID NO:21)
LONG-N- : TTAACTCAAAGCTCTACATCATTATCTTTTGG (SEQ ID NO:22)

Les produits de PCR ont été digérés par *Bam*HI et clonés dans le plasmide pGEX-4T-3 aux sites *Bam*HI-*Sma*I. La région codant pour N a été sous-clonée par digestion du plasmide pGEX-N par *Bam*HI*-Xho*I et sous-clonée dans le plasmide pET28a+ (Novagen).

### • Mutagenèse du plasmide pET-N et création d'un site de restriction unique Sal en amont du codon STOP

Un site de restriction *SacI* a été introduit juste avant le codon stop de la protéine N du RSV humain (souche long) par mutagenèse dirigée en utilisant les oligonucléotides :
Nfinsac+ CCAAAAGATAATGATGTAGAGCTCTGACTCGAGCACCACCACC (SEQ ID NO: 23)
Nfinsac- GGTGGTGGTGCTCGAGTCAGAGCTCTACATCATTATCTTTTGG (SEQ ID NO: 24)

Ceci a permis d'obtenir le plasmide pET-N-Sac.

### • Obtention de la protéine N fusionnée à la GFP

La GFP a été sortie du plasmide pEGFPN1 par digestion par l'enzyme de restriction *Eagl,* puis traitement à la Klenow en présence de nucléotides pour obtenir des bouts francs sur l'ADN, puis digéré par *SacI.* Le plasmide pET-N-Sac a été digéré par l'enzyme *XhoI,* traité à la Klenow en présence de nucléotides puis digéré par Sal. La GFP a ainsi été insérée dans le plasmide pET-N-Sac et a permis d'obtenir le plasmide pET-N-GFP

### • Obtention de la protéine N-épitopes F

Des épitopes « mimotope » et « heptad » de la protéine F du RSV ont été clonés en C-terminal de la protéine N par insertion d'oligonucléotides double brins.

### Epitope Mimotope :

F-Flag-HWSISKPO+ :
   CATGGACTACAAGGACGACGATGACAAGCACTGGTCTATCTCTAAACCGCAGTAG (SEQ ID NO: 25)
F-Flag-HWSISKPQ- :

Ces oligonucléotides hybridés entre eux ont été insérés aux sites *SacI SalI* dans le plasmide pET-N-GFP.

### Epitope Heptad.

Les oligonucléotides suivants ont été hybridés entre eux :
FheptadAge+ : CCGGTCTCTACTAACAAAGCTGTTGTTTCTCTGAGCTAGT (SEQ ID NO: 27) (STNKAVVSLS) (SEQ ID NO: 28)
FheptadAge- : CCGGACTAGCTCAGAGAAACAACAGCTTTGTTAGTAGAGA (SEQ ID NO: 29)
Ils ont été clonés au site *Agel* dans le plasmide pET-N-GFP

### • Clonage de P231-241

Les amorces suivantes ont été dénaturées par chauffage à 94°C, pendant 5 minutes, puis refroidies à température ambiante :
P231NB+: GATCCGATAGTGACAATGATCTATCACTTGAAGATTTCTGA (SEQ ID NO:30)
P231 N- :TCAGAAATCTTCAAGTGATAGATCATTGTCACTATCG (SEQ ID NO:31)

Après hybridation, 10 ng d'oligonucléotides double brin ont été ligaturés avec 100 ng d'ADN de plasmide pGEX-4T-3 digéré par les *enzymes Bam*HI et *Smal* et purifié par électrophorèse en gel d'agarose. Les plasmides recombinants ont été vérifiés par séquençage au niveau du gène N.

### Exemple 2 : Expression et purification des complexes

Des bactéries BL21 (DE3) (Novagen) compétentes ont été transformées avec 1 µg d'ADN pGEX-PΔ et 1 μg d'ADN pET-N puis étalées sur boîte de Pétri contenant du milieu LB-agar supplémenté avec 100 μg/ml final d'ampicilline et 50 μg/ml final de Kanamycine. Une colonie a été repiquée et mise à pousser une nuit à 37°C dans 2 ml de milieu LB contenant Ampicilline et Kanamycine à 100 μg/ml et 50 μg/ml respectivement. Le lendemain matin 1 ml de culture saturée a servi à ensemencer 1 litre de milieu LB additionné des antibiotiques et mis à pousser jusqu'au soir. Le soir, un volume de milieu frais LB contenant de l'IPTG (qui induit l'expression des protéines) à une concentration de 160 μg/ml est ajouté à la culture et le tout est mis à pousser sur la nuit à 28°C. Le lendemain, les bactéries sont centrifugées 15 minutes à 5000 tpm et le culot est repris dans 100 ml du tampon suivant :
50 mM Tris pH 7,8
60 mM NaCl
2 mM DTT
1 mM EDTA
4 mM Benzamidine
Antiprotéases 1X (Complete EDTA-free protease inhibitor cocktail, ref. Roche n° 11 873 580 001) soit une tablette pour 50 ml de tampon de lyse
0,1% Triton-X100

10 ml du même tampon additionné de Lysozyme à 10mg/ml (1mg/ml final) sont ajoutés. Les bactéries sont incubées 1 heure dans la glace (lyse). Quand le tout devient visqueux, l'ensemble est soniqué 3 fois 1 minute en utilisant une sonde plongeant dans le mélange, dans la glace en laissant reposer 5 minutes entre chaque sonication. L'ensemble est centrifugé 30 minutes à 10 000 g à 4°C puis le surnageant est récupéré. Le surnageant est recentrifugé 30 minutes à 10 000 g à 4°C puis le nouveau surnageant est récupéré. 4 ml de billes de sépharose 4B-glutathion (Amersham-Pharmacia) sont lavés en prélevant 8 ml de mélange billes tampon (vol/vol) avec le tampon de lyse. Les billes sont laissées dans un volume équivalent de tampon, ajoutées au lysat bactérien clarifié, et mises à tourner à 4°C sur la nuit. Le lendemain les billes sont centrifugées à 2000 rpm pendant 3 minutes, puis le surnageant est éliminé, et les billes lavées trois fois avec le tampon de lyse dépourvu d'antiprotéases, trois fois en tampon PBS 1X.

Les billes son clivées au site thrombine en utilisant de la thrombine biotinylée (Novagen) à raison d'1 µl (1 U) de thrombine ("Thrombin Cleavage Capture Kit", Novagen N° 69022-3FRZ) pour 1 ml de billes. Les billes sont incubées sur la nuit à 20°C et, le lendemain, centrifugées 3 minutes à 2000 rpm, laissées à décanter 15 minutes pour récupérer le surnageant. Un volume équivalent de PBS 1X est ajouté sur les billes ; le mélange est agité, laissé décanter. Le surnageant est à nouveau récupéré et rajouté au surnageant prélevé précédemment. Au surnageant récupéré sont ajoutées des billes d'agarose Streptavidine (Novagen ref. 69203) à raison de 16 µl de résine (soit 32 µl de mélange résine/tampon (vol/vol). Le mélange est laissé sous agitation pendant une heure, puis centrifugé 3 minutes à 2000 rpm et le surnageant est récupéré. On obtient une concentration en protéines de 2 mg/ml.

10 µl du surnageant contenant les produits de clivage sont dénaturés en tampon de Laemmli 1X, mis à bouillir et déposés sur un gel de polyacrylamide à 12% en tampon Tris-Glycine SDS 0,1%, puis colorés au bleu de Coomassie après électrophorèse pour visualiser les protéines.

### Exemple 3 : Séparation de N-GFP et PΔ161N (P161-241) et purification des anneaux de N-GFP

Les protéines présentes dans le surnageant peuvent être séparées par chromatographie d'exclusion de taille (gel filtration) en PBS 1X.

### Exemple 4 : Capture des complexes N-PΔN par différentes lignées cellulaires : étude à l'aide de la protéine de fusion N-Green Fluorescent Protein (GFP).

### I. Matériel et méthodes

### I.1 Lignées cellulaires et tests de phagocytose:

- HEp-2 : lignée épithéliale humaine de larynx (Cancer Res 1955;15:598), cultivées en monocouche en EMEM (Eagle's Minimum Essential Medium) + 10% SVF+ L-glutamine+ PS (pénicilline, streptomycine).
- RAW : lignée de monocytes-macrophages péritonéaux murins (J. Immunol 1977;119:950) cultivée en monocouche en DMEM (Dulbecco's Minimum Essential Medium) + 10% SVF + L-glutamine + PS.

Les lignées cellulaires sont dissociées en D-PBS (Dulbecco's Phosphate Buffered Saline) + 0,33% lidocaïne + 5 mM EDTA. Les cellules isolées sont lavées, reprises à une concentration de 10⁷ cellules/ml en PBS + 2%SVF et incubées sous agitation douce dans des tubes de 5 ml à fond rond en présence de :
- P161-241 + N-GFP (N-GFP-PΔN) à 20 μg/ml
- GFP à 6,5 μg/ml
- Dextran-FITC (DX-FITC) à 1 mg/ml (Ref : D-1844, Molecular Probes).

Les incubations sont faites en parallèle à 4°C, température à laquelle les mécanismes d'endocytose sont inhibés et à 37°C, température permissive pour l'internalisation. Après une heure ou deux heures d'incubation, les cellules sont lavées trois fois et reprises en PBS + 2%SVF

### I.2 Analyse de l'adsorption/internalisation par cytométrie en flux

On compare, par cytométrie en flux (FACSCalibur, Becton), la fluorescence (FL1) associée aux cellules (HEp-2, RAW) incubées avec des molécules fluorescentes (DX-FITC ou N-GFP-PΔN) à 4°C ou 37°C. Le niveau d'autofluorescence des cellules est donné par des cellules incubées deux heures à 37°C en l'absence de molécules fluorescentes.

### I.3 Analyse de l'adsorption/internalisation en microscopie à fluorescence

Après incubation, 10⁵ cellules sont déposées par cytocentrifugation sur des lames superfrost plus (SFPLUS-42, Milian). Les lames sont séchées 15 minutes et montées en PERTEX (Ref : 00814, Histolab).

Le niveau de fluorescence est regardé au microscope (Axiovert200M, Zeiss). La prise de vues est réalisée avec une caméra (Coolsnap HQ) en utilisant le logiciel Metavue.

### I.4 Immunodétection de la protéine N après perméabilisation des cellules.

L'objectif est de comparer, par immunodétection de la protéine N, le signal détecté sur les cellules non perméabilisées (complexe N-GFP-PΔN extracellulaire), au signal détecté sur cellules perméabilisées (complexe N-GFP-PΔN intra et extracellulaire).

Les cellules sont fixées pendant 15 minutes en tampon A (Ref : GAS-003, Caltag) et lavées.

Dans le cas des cellules non perméabilisées, le marquage se fait en tampon Cell Wash (Becton) +5%SVF. Pour perméabiliser les cellules pendant le marquage, le tampon B (Ref : RGAS-003, Caltag) est employé. Les récepteurs Fc sont saturés pendant 20 minutes par un anticorps anti-CD16/CD32 (Ref : 553141, Becton) dilué au 1/100. Puis les cellules sont incubées 30 minutes avec un sérum de lapin dirigé contre la protéine N, dilué au 1/1000. La détection des Ig de lapins se fait par une incubation de 30 minutes avec un anticorps biotinylé (Ref: BA-1000, Vector) dilué au 1/200. L'utilisation de streptavidine couplée à l'APC (554067, Becton) au 1 /500 permet la détection indirecte sur le canal FL4 de la protéine N en cytométrie en flux (FACSCalibur, Becton).

### II. Résultats :

### II-1 Analyse de l'interaction des complexes N-GFP-PΔN avec les cellules RAW (macrophages) et HEp2 (épithéliales):

Les cellules RAW sont une lignée de monocytes-macrophages. Ils ont une capacité de phagocytose que l'on peut observer en mettant à leur contact un polymère fluorescent, le DX-FITC. La capture du DX-FITC est fortement augmentée à 37°C par rapport à 4°C (fig. 1B). La lignée HEp-2 est une lignée épithéliale. Elle n'a pas de capacité de phagocytose, c'est pourquoi la fluorescence liée à l'interaction entre le DX-FITC et ces cellules est peu différente entre les incubations à 4°C et à 37°C (fig. 1 A).

Lorsque les complexes N-GFP-PΔN sont mis en contact avec les cellules RAW et HEp-2 on observe une forte augmentation du niveau de fluorescence par rapport au niveau basal (PBS) (fig. 1 C, D). Cela indique une adsorption efficace à la membrane de ces cellules. Après incubation à 37°C, la fluorescence détectée en présence de N-GFP-PΔN diminue dans les deux types cellulaires, mais reste néanmoins supérieure au niveau basal (fig. 1 C, D). Lorsque les cellules sont incubées avec de la GFP recombinante, le signal de fluorescence reste au niveau basal (données non montrées). Ceci indique que les phénomènes de capture observés sont liés aux anneaux de N-PΔN et non à la GFP.

La capture des anneaux de N-GFP-PΔN□ dans les lignées RAW et HEp-2 a été confirmé en microscopie.

La fluorescence spécifique de la GFP fusionnée à la protéine N est retrouvée sous forme de granules à l'intérieur des cellules HEp-2 ou RAW. Cette fluorescence n'est pas retrouvée dans le cas où l'incubation est faite avec de la GFP recombinante. Les complexes N-GFP-PΔN sont donc adsorbés et/ou internalisés dans la lignée macrophagique RAW et dans la lignée épithéliale HEp-2.

### II.2 Internalisation des complexes N-GFP-PΔN par les cellules RAW

Afin de comprendre le devenir des complexes N-GFP-PΔN dans les cellules RAW à 37°C versus 4°C, les cellules sont perméabilisées et la présence de la protéine N est révélée par immunomarquage et analyse en cytométrie en flux (fig. 2). En absence de perméabilisation, la quantité de N détectée (signal de fluorescence) est plus faible à 37°C qu'à 4°C (fig. 2A). En revanche, après perméabilisation, le niveau du signal redevient identique entre les conditions d'incubation 37°C et 4°C (fig. 2B). Les complexes N-GFP-PΔN sont donc internalisés lors de l'incubation à 37°C.

En conclusion, l'ensemble des expériences réalisées avec les complexes N-GFP-PΔN, en cytométrie en flux et en microscopie, a permis de mettre en évidence des propriétés originales de ces structures protéiques. Les assemblages en anneaux de la nucléoprotéine ont la capacité de s'adsorber et d'être internalisés efficacement par des types cellulaires variés comme les macrophages, les cellules épithéliales et les cellules dendritiques (données non montrées). Ces propriétés sont particulièrement intéressantes pour l'utilisation de ces structures comme vecteur d'antigène en vaccination.

### Exemple 5 : Evaluation des propriétés de vecteur antigénique de la protéine N en anneau vis à vis de la Green Fluorescent Protein (GFP) exprimée en fusion

### I. Matériel et méthodes

### I.1 Souris :

BALB/c femelles de 8-10 semaines, élevées à l'Unité Expérimentale Animalerie Rongeur (INRA, Jouy-en-Josas).

### I.2 Antigènes :

- P161-241 + N-GFP (N-GFP-PΔN) concentration à 0,1064 μg/μl
- GFP concentration à 0,5 μg/μl
- P161-241+N (N-PΔN) concentration à 1 μg/μl

### I.3 Adjuvant :

- LT(R192G) lymphotoxine détoxifiée de *E. Coli,* 1 mg/ml (lot envoyé par John Clements ; Choi et al., 2004, Protein Expression and Purification 38, pp 205)

### I.4 Immunisation:

Administration par voie intra-nasale (i.n.) sous anesthésie avertine (300 □l i.p.) du mélange suivant (60 μl / souris) :
- 5 μg de LT(R192G)
- 5 μg N-GFP-PΔN
   ou 1.7 μg de GFP soluble
   ou 3.3 μg N-PΔN
   (quantités en GFP ou en N-PΔN correspondantes à celles présentes dans 5 μg de N-GFP-PΔN)
- qsp 60 μl avec sérum physiologique apyrogène

Toutes les solutions sont passées sur filtre 0,22 μm avant injection.

| Groupes | J0 Primo-injection | J14 rappel | J32 autopsie |
|---|---|---|---|
| | LT(R192G) | LT(R192G) | Serum |
| **témoin** | i.n. | i.n. | Rate |
| | serum | serum | LBA |
| | | | gg drainants |
| | GFP | GFP | Serum |
| **GFP** | +LT(R192G) | + LT(R192G) | Rate |
| | i.n. | i.n. | LBA |
| | serum | serum | gg drainants |
| | N-PΔN | N-PΔN | Serum |
| | + LT(R192G) | + LT(R192G) | Rate |
| **N-PΔN** | i.n. | i.n. | LBA |
| | serum | serum | gg drainants |
| | NGFP-PΔN | NGFP-PΔN | Serum |
| **N-GFP-PΔN** | + LT(R192G) | + LT(R192G) | Rate |
| | **i.n.** | **i.n.** | LBA |
| | serum | serum | gg drainants |

| | | | |
|---|---|---|---|
| LBA: lavage broncho-alvéolaire (avec 1 ml de PBS 1 mM EDTA) gg: ganglions | | | |

### I.5 Production d'anticorps anti N-PΔN et anti GFP :

Les anticorps anti-N-PΔN ou anti-GFP (IgH+L et IgA) sont recherchés dans les sérums et les LBA par ELISA :
- Les sérums sont collectés à partir des prélèvements de sang (1 nuit d'exsudation à 4°C) puis congelés à -20°C.
- Les LBA sont centrifugés 5 min à 1700 rpm, les surnageants sont collectés (environ 1 ml) et congelés à -20°C.

Des plaques de 96 puits (Immulon 2HB, ThermoLabsystems) sont sensibilisées une nuit à 4°C avec le complexe N-PΔN ou la protéine GFP recombinante (200 ng par puit, 100 μl par puit) en tampon bicarbonate 0,1 M pH 9,5. Les plaques sont lavées 5 fois avec 200 μl par puit de PBS 0,05% tween 20 (utilisation d'un automate Wellwash, Labsystems). Les plaques sont ensuite saturées pendant 1 h à 37°C avec 150 μl par puit de tampon PBS 0,05% tween 20 et 5% sérum de veau foetal (PBS-T-SVF). Après 5 lavages, les échantillons à titrer sont dilués en PBS-T-SVF (sept dilutions successives de raison 3 en partant d'une première dilution au 30^{ième}pour les sérums et au tiers pour les LBA). Les plaques sont incubées pendant 2h à 37°C. Après 5 lavages, l'anticorps secondaire dilué en PBS-T-SVF est distribué à raison de 100 µl par puit. Les anticorps secondaires utilisés sont conjugués à la peroxydase et dirigés contre les immunoglobulines de souris : Ig(H+L) (4000^{ième}, P.A.R.I.S.) ou IgA (1000^{ième}, Caltag). Les plaques sont incubées pendant 2 h à 37°C et lavées 5 fois. Les plaques sont ensuite incubées avec le substrat de la peroxydase (TMB, 100 µl par puit) pendant 10 min à l'obscurité. La réaction enzymatique est arrêtée par l'ajout de 50 µl de H₃PO₄ 2M. Les densités optiques (DO) sont lues à 450 nm (lecteur Dynex). La courbe D0₄₅₀=f(dilution) est modélisée par la courbe de régression y=(b+cx)/(1+ax) à l'aide du logiciel Origin. Le titre en anticorps est déterminé comme la valeur de dilution donnant deux fois la D0₄₅₀ d'un échantillon témoin (J0) à sa plus grande dilution.

### I.6 Production d'IFN-γ/IL-5/IL-10 par des lymphocytes T spécifiques de N-PΔN ou de GFP

La rate et les ganglions drainant le tractus respiratoire (faciaux, cervicaux et médiastinaux) sont traités selon le même protocole. Les rates sont traitées individuellement et les ganglions sont groupés par lot expérimental.

Les organes lymphoïdes sont émincés puis broyés délicatement sur filtre (tamis cellulaire 100 μm, BD Falcon) en milieu RPMI et PS. La suspension cellulaire est centrifugée à 1700 rpm pendant 10 min à 4°C. Les cellules sont remises en suspension dans 1 ml de tampon de lyse des érythrocytes (tampon salin hypotonique) et incubées pendant 5 min à température ambiante. La réaction de lyse est arrêtée par ajout de 10 ml de RPMI complet (PS, 2 mM L-glutamine et 10% SVF). Les débris membranaires sont décantés et les cellules sont lavées trois fois par centrifugation (1700 rpm pendant 10 min à 4°C). Les suspensions cellulaires sont dénombrées à l'aide d'une cellule de Malassez.

Les cellules sont mises en culture dans des microplaques de 96 puits traitées pour la culture cellulaire (Falcon) à raison de 400 000 cellules par puit dans 200 μl de milieu RPMI complet. Quatre conditions de culture sont testées en triplicat sur chaque suspension cellulaire :
- PMA (Phorbol 12-myristate 13-acétate, Sigma) 10 ng/ml et ionomycine (Sigma) 1 μg/ml (témoin positif, activation polyclonale)
- RPMI complet (témoin négatif)
- N-PΔN 10 μg/ml
- GFP 10 μg/ml.

Après 48h (IL-5 et IL-10) ou 72h (IFN-γ) de culture à 37°C avec 5% CO₂, les surnageants de culture sont collectés et congelés à -20°C jusqu'à titration des cytokines par ELISA :
Des plaques de 96 puits (Immulon 2HB, ThermoLabsystems) sont sensibilisées une nuit à 4°C avec l'anticorps de capture anti-cytokine de souris à 4 μg/ml (IFN-γ) ou 2 μg/ml (IL-5/IL-10) en tampon Bicarbonate 0,1 M pH 9,5 (100 μl/puit). Les plaques sont lavées 5 fois avec 200 μl par puit de PBS 0,05% tween 20 (utilisation d'un automate Wellwash, Labsystems). Les plaques sont ensuite saturées pendant 2 h à 37°C avec 150 μl par puit de tampon PBS 0,05% tween 20 et 2% d'albumine sérique bovine (PBS-T-BSA). Après 5 lavages, la référence cytokine de souris recombinante et les échantillons à titrer sont dilués en PBS-T-BSA par dilutions successives au demi. Quatre dilutions successives au demi sont réalisées sur les échantillons purs. La plaque est alors incubée pendant une nuit à 4°C. Après 5 lavages, l'anticorps de détection biotinylé est distribué (1 μg/ml pour IFN-γ/IL-10 ou 0,5 μg/ml pour IL-5 en PBS-T-BSA, 100 μl/puit) et incubé 3 h à 4°C. Après 5 lavages, le conjugué Streptavidine-Peroxydase (Pierce) est distribué (1μg/ml en PBS-T-BSA, 100 μl/puit) et incubé 1 h à 4°C. Après 5 lavages, le substrat de la peroxydase (ABTS+H₂O₂) est distribué dans les puits. Après 45 minutes d'incubation, les densités optiques sont lues à 405 nm (Lecteur ELISA Dynex). Le calcul de la concentration en IFN-γ/IL-5/IL-10 dans les échantillons se fait par rapport à la gamme étalon en utilisant le logiciel d'analyse « Révélation » couplé au lecteur.

Références des anticorps (BD Bioscience) :
- IFNγ : anticorps de capture : clone R4-6A2 (BD Bioscience, 551216)
- Anticorps de détection : clone XMG1.2 (BD Bioscience, ref 554410)
- mIFN-γ recombinant : R&D systems 485-MI
- IL-5 : anticorps de capture : clone TRFK5 (BD Bioscience, 554393)
- Anticorps de détection : clone TRFK4 (BD Bioscience, ref 554397)
- mIL-5 recombinant : BD Bioscience 554581
- IL-10 : anticorps de capture : clone JES5-2A5 (BD Bioscience, 551215)
- Anticorps de détection : clone SXC-1 (BD Bioscience, ref 554423)
- mIL-10 recombinant : BD Bioscience 550070
- Streptavidin peroxydase Immunopure (Pierce 21126)

### I.7 Analyses statistiques :

Les groupes sont comparés deux par deux en réalisant un test U de Mann-Whitney (http://eatworms.swmed.edu/∼leon/stats/utest.html). Une valeur p<0,05 est considérée significative.

### II. Résultats :

Afin d'évaluer les potentialités des anneaux formés par le complexe N-PΔN comme vecteur vaccinal, des souris BALB/c ont été immunisées contre la GFP (antigène modèle) sous la forme d'une protéine de fusion avec la nucléoprotéine N du RSV. La protéine de fusion N-GFP forme des structures en anneaux solubles qui sont purifiables selon le procédé décrit précédemment (brevet FR0504426). On obtient ainsi des complexes protéiques NGFP-PΔN. Pour une utilisation vaccinale, les protéines N-GFP et P peuvent être séparées mais cette opération n'est pas nécessaire, les résultats antérieurs des Inventeurs sur l'immunogénicité des anneaux de N ayant montré que la présence de PΔN n'a pas d'effet négatif.

Compte tenu des travaux orientés sur les pathogènes ciblant les voies respiratoires et de l'immunogénicité démontrée des complexes N-PΔN administrés par voie nasale, cette voie d'administration a été privilégiée pour démontrer les propriétés vectrices de ces complexes. Des lots de souris ont été également immunisées contre la protéine GFP sous sa forme recombinante native et contre les complexes N-PΔN. La lymphotoxine détoxifiée de *E. Coli,* LT(R192G) dont les propriétés adjuvantes par voie mucosale ont été bien décrites (McNeal et al. 2002, Freytag et Clements 2005) a été utilisée comme adjuvant pour tous les groupes d'animaux de cet exemple.

Les paramètres de la réponse immunitaire dirigée contre la GFP et contre N-PΔN □qui ont été suivis sont (i) la production d'anticorps sériques et muqueux (par lavages broncho-alvéolaires) et (ii) la réponse cellulaire via la production de cytokines (IFN-γ, IL-5 et IL-10) par des lymphocytes T mémoires isolés de la rate ou des ganglions drainant le tractus respiratoire.

### II-1 Les anneaux de N sont un vecteur efficace pour cibler des anticorps contre un antigène exogène, exemple de la GFP

Les complexes N-PΔN sont très fortement immunogènes par voie systémique et par voie mucosale (brevet FR0504426). La fusion de la GFP avec la protéine N n'altère pas l'intensité de la réponse Ac anti N-PΔN, que ce soit au niveau sérique (fig. 3B) et mucosal (fig. 3C) : il n' y a pas de différence significative entre les groupes N-GFP-PΔN et N-PΔN.

La GFP administrée par voie nasale induit une réponse Ac systémique (fig. 3A ;GFP versus témoin, p=0,003) mais pas de réponse locale (fig. 3C ; GFP versus témoin, non significatif).

Par contre, l'association de la GFP aux complexes N-PΔN augmente fortement le taux d'anticorps anti-GFP par rapport à la GFP seule. Le niveau des anticorps anti-GFP est multiplié par un facteur 4 au niveau sérique (N-GFP-PΔN versus GFP, p=0,039) et par un facteur 100 au niveau des LBA (N-GFP-PΔN versus GFP, p=0,014).

### II-2 La vectorisation de la GFP sur les anneaux de N n'augmente pas la réponse T spécifique :

En plus de leur forte capacité à stimuler la réponse anticorps, les N-PΔN stimulent efficacement la réponse T mémoire mise en évidence par une synthèse d'IFN-γ antigène-dépendante au niveau systémique (brevet FR0504426 et Figure 4, rates N-PΔN versus témoin, p=0,01) mais également locale (fig. 4, ganglions loco-régionaux). La fusion d'un antigène exogène tel que la GFP sur la N ne perturbe pas l'établissement de ces réponses T que ce soit au niveau de la rate (N-GFP-PΔN versus N-PΔN, non différent) ou au niveau des ganglions (Figure 4).

Par contre, quel que soit le mode de présentation de la GFP (seule ou fusionnée à N) aucune réponse T spécifique de la GFP, mesurée par la production d'IFN-γ (Fig. 4) ou d'IL-5/IL-10 (non montré) n'est observée : il n'y a pas de différence significative entre les groupes témoin, N-GFP-PΔN et GFP.

En conclusion, l'exemple de la GFP a permis de montrer que les complexes N-PΔN sont des vecteurs d'antigène très puissants pour stimuler des réponses anticorps, en particulier au niveau mucosal. Par contre ils s'avèrent peu efficaces pour stimuler une réponse T mémoire contre ces mêmes antigènes. Il est important de noter que la fusion d'un antigène exogène n'a pas altéré l'immunogénicité propre aux complexes N-PΔN.

Ces résultats permettent d'envisager l'utilisation des complexes N-PΔN comme vecteur d'antigène dans le but de stimuler de fortes réponses anticorps. Dans le cadre de la vaccination contre le RSV, les résultats obtenus montrent que l'on peut tirer avantage à la fois de la réponse cellulaire contre la nucléoprotéine et d'une réponse anticorps contre des antigènes/épitopes du RSV connus pour être la cible des anticorps neutralisants. Ces deux facettes des défenses immunitaires sont critiques pour obtenir une immunité stérilisante contre le RSV.

De manière générale, pour de nombreux pathogènes (en particulier les virus), les épitopes ou antigènes cibles des anticorps neutralisants sont bien décrits dans la littérature et participent au contrôle efficace de l'infection. De nouveaux vecteurs pour stimuler efficacement ces réponses sont donc très recherchés en vaccination. Les complexes N-PΔN présentent en plus l'avantage d'être des structures inertes non réplicatives donc sans risques pour l'hôte et l'environnement.

### Exemple 6 : Optimisation de la séquence de liaison entre la protéine N et la protéine GFP

La purification des protéines de fusion N-GFP est effectuée par affinité avec la protéine P
(fragment 161-241) fusionnée à la GST. La protéine N-GFP construite conformément à l'exemple 1 (SEQ ID NO: 33) se purifie avec un rendement dix fois plus faible que la protéine N. De plus, on observe toujours la présence de deux polypeptides migrant avec une masse apparente d'environ 43 kDa. Ces bandes ont été analysées par spectrométrie de masse (MALDI-TOF). Elles correspondent à un clivage qui a lieu (i) dans la séquence de liaison séparant N de GFP (au niveau d'une arginine) résultant en l'addition de 11 résidus en C-terminal de la protéine N, et (ii) dans la partie C-terminale de la protéine N (au niveau d'une lysine) en enlevant les 6 derniers résidus. La construction d'une protéine N délétée de 6, 12 et 27 résidus en C-terminal a montré que les protéines NΔ6C et NΔ12C interagissaient toujours avec P, contrairement à la forme tronquée NΔ27C.

La séquence de liaison entre N et GFP a donc été modifiée, en remplaçant la séquence KLRILQSTVPR↓ARDPPVAT (SEQ ID NO: 34, la flèche indique le site de clivage chez *E. coli*) par la séquence KLRILQSTVPSERPQASGVYMGNLTTRGPVAT (SEQ ID NO: 32).

Ceci a résulté en une protéine de fusion N-GFP (SEQ ID NO: 35) plus stable, qui n'est plus dégradée dans la bactérie (absence de bandes supplémentaires en gel SDS-PAGE) et qui se purifie avec les mêmes rendements que la protéine N native, seule.

## Revendications

1. Procédé de préparation d'un complexe protéine N-protéine d'intérêt/protéine P, les protéines N et P étant des protéines d'un virus respiratoire syncytial (RSV), ledit procédé comprenant les étapes consistant à :
a) coexprimer une protéine de fusion protéine N d'un virus respiratoire syncytial - protéine d'intérêt, avec une protéine P tronquée du même virus respiratoire syncytial,
dans laquelle la protéine d'intérêt est fusionnée en phases à l'extrémité C-terminale de la protéine N, la protéine N étant une protéine native ou une protéine N modifiée dans la région définie par les 25 derniers acides aminés C-terminaux, ladite protéine N conservant la capacité d'interagir avec la protéine P,
la protéine P tronquée ne contenant pas le domaine d'oligomérisation P et comprenant un domaine de liaison à la protéine N;
b) recueillir les complexes protéine N-protéine d'intérêt/protéine P ainsi formés.

2. Procédé selon la revendication 1, dans lequel le virus respiratoire syncytial est le virus respiratoire syncytial humain.

3. Procédé selon la revendication 1 ou 2, dans lequel la protéine P tronquée est exprimée sous forme d'une protéine de fusion avec une protéine facilitant la purification des complexes N-protéine d'intérêt/protéine P.

4. Complexe protéine N-protéine d'intérêt/protéine P sous forme d'anneaux solubles comprenant la protéine de fusion protéine N-protéine d'intérêt avec leur ARN, les protéines N et P étant des protéines d'un virus respiratoire syncytial, dans lequel la protéine d'intérêt est fusionnée en phase à l'extrémité C-terminale de la protéine N,
la protéine N étant une protéine native ou une protéine N modifiée dans la région définie par les 25 derniers acides aminés C-terminaux, ladite protéine N conservant la capacité d'interagir avec la protéine P,
la protéine P étant une protéine P tronquée du même virus respiratoire syncytial ne contenant pas le domaine d'oligomérisation P et comprenant un domaine de liaison à la protéine N.

5. Complexe protéine N-protéine d'intérêt/protéine P selon la revendication 4, dans lequel la protéine d'intérêt est une protéine marqueur, ou une protéine d'intérêt vaccinal.

6. Complexe protéine N-protéine d'intérêt/protéine P selon la revendication 5, dans lequel la protéine d'intérêt vaccinal est un antigène dérivé d'un microorganisme pathogène.

7. Complexe protéine N-protéine d'intérêt/protéine P selon la revendication 5, dans lequel la protéine d'intérêt est une construction comprenant la GFP fusionnée en phase, à son extrémité C-terminale, avec une protéine vaccinale.

8. Complexe protéine N-protéine d'intérêt/protéine P selon la revendication 4, dans lequel la protéine de fusion protéine N-protéine d'intérêt est une protéine de fusion N-GFP-protéine d'intérêt comprenant la GFP (Green Fluorescent Protein) fusionnée en phase à l'extrémité C-terminale de la protéine N, incluant une séquence liaison entre les protéines N et GFP ayant pour séquence SEQ ID NO: 32.

9. Procédé de préparation d'une protéine N d'un virus respiratoire syncytial, fusionnée en phase à son extrémité C-terminale avec une protéine d'intérêt, ledit procédé comprenant la séparation de la protéine de fusion protéine N-protéine d'intérêt à partir du complexe protéine N-protéine d'intérêt/protéine P selon l'une quelconque des revendications 4 à 8.

10. Protéine de fusion protéine N-protéine d'intérêt, isolée, structurée en anneaux solubles comprenant la protéine de fusion protéine N-protéine d'intérêt avec leur ARN, susceptible d'être obtenue par le procédé selon la revendication 9, où la protéine N est une protéine N d'un virus respiratoire syncytial native ou modifiée dans la région définie par les 25 derniers acides aminés C-terminaux, et la protéine d'intérêt est fusionnée en phase, à l'extremité C-terminale de la protéine N.

11. Composition pharmaceutique comprenant une protéine N d'un virus respiratoire syncytial fusionnée avec une protéine d'intérêt telle que définie dans la revendication 10 ou un complexe protéine N-protéine d'intérêt/protéine P tel que défini dans l'une quelconque des revendications 4 à 8, et un véhicule pharmaceutiquement acceptable.

12. Composition selon la revendication 11 qui est une composition immunogène ou vaccinale.

13. Réactif diagnostic comprenant une protéine de fusion selon la revendication 10, dans laquelle la protéine d'intérêt est un antigène.

14. Utilisation d'une protéine de fusion selon la revendication 10 comprenant un antigène fusionné en phase à l'extrémité C-terminale d'une protéine N d'un virus respiratoire syncytial pour la détection *in vitro* d'anticorps dirigés contre ledit antigène de la protéine de fusion.

15. Méthode de détection d'anticorps spécifiques d'un antigène, dans un échantillon biologique, comprenant les étapes consistant à :
a) mettre en contact ledit échantillon biologique avec une protéine de fusion selon la revendication 10, comprenant un antigène fusionné en phase à l'extrémité C-terminale d'une protéine N d'un virus respiratoire syncytial,
b) détecter les complexes protéine de fusion N-antigène/anticorps formés,
la présence de tels complexes étant révélatrice de la présence d'anticorps spécifiques de l'antigène dans l'échantillon biologique.

## Patentansprüche

1. Verfahren zur Herstellung eines Komplexes Protein N-Protein von Interesse /Protein P, wobei die Proteine N und P Proteine eines Respiratorischen Syncytial-Viruses (RSV) sind, wobei das Verfahren die Schritte umfasst bestehend aus:
a) Co-Exprimieren eines Fusionsproteins Protein N eines Respiratorischen Syncytial-Viruses - Protein von Interesse, mit einem geschnittenen Protein P aus dem selben Respiratorischen Syncytial-Virus,
wobei das Protein von Interesse an das C-terminale Ende des Proteins N fusioniert ist, wobei das Protein N ein natives Protein oder ein in dem durch die letzten 25 C-terminalen Aminosäuren definierten Bereich modifiziertes Protein N ist, wobei das Protein N die Fähigkeit beibehält, mit dem Protein P zu interagieren,
wobei das geschnittene Protein P keine Oligomerisationsdomäne P enthält und eine Domäne zur Bindung an das Protein N umfasst;
b) Sammeln des so gebildeten Komplexes Protein N-Protein von Interesse/Protein P.

2. Das Verfahren gemäß Anspruch 1, wobei das Respiratorischen Syncytial-Virus das humane Respiratorische Syncytial-Virus ist.

3. Das Verfahren gemäß Anspruch 1 oder 2, wobei das geschnittene Protein P in Form eines Fusionsproteins mit einem Protein exprimiert wird, das die Aufreinigung der Komplexe N-Protein von Interesse /Protein P erleichtert.

4. Komplex aus Protein N-Protein von Interesse /Protein P in Form löslicher Ringe umfassend das Fusionsprotein Protein N-Protein von Interesse mit seiner RNA, wobei die Proteine N und P Proteine eines Respiratorischen Syncytial-Viruses sind, wobei das Protein von Interesse an das C-terminale Ende des Proteins N fusioniert ist,
wobei das Protein N ein natives Protein oder ein in dem durch die 25 letzten C-terminalen Aminosäuren definierten Bereich modifiziertes Protein N ist, wobei das Protein N die Fähigkeit beibehält, mit dem Protein P zu interagieren,
wobei das Protein P ein geschnittenes Protein P des selben Respiratorischen Syncytial-Viruses ist, das keine Oligomerisationsdomäne P enthält und eine Domäne zur Bindung an das Protein N umfasst.

5. Der Komplex Protein N-Protein von Interesse /Protein P gemäß Anspruch 4, wobei das Protein von Interesse ein Markerprotein oder ein Protein, das für die Impfung von Interesse ist, ist.

6. Der Komplex Protein N-Protein von Interesse /Protein P gemäß Anspruch 5, wobei das Protein von Impf-Interesse ein von einem pathogenen Mikroorganismus abgeleitetes Antigen ist.

7. Der Komplex Protein N-Protein von Interesse /Protein P gemäß Anspruch 5, wobei das Protein von Interesse ein Konstrukt umfassend das über sein C-terminales Ende mit einem Impf-Protein fusioniertes GFP ist.

8. Der Komplex Protein N-Protein von Interesse /Protein P gemäß Anspruch 4, wobei das Fusionsprotein Protein N-Protein von Interesse ein Fusionsprotein N-GFP-Protein von Interesse umfassend an das C-terminale Ende des N-Proteins fusioniertes GFP (Green Fluorescent Protein) ist beinhaltend eine Bindungssequenz zwischen den Proteinen N und GFP mit der Sequenz SEQ ID NO: 32.

9. Verfahren zur Herstellung eines Proteins N eines Respiratorischen Syncytial-Viruses, das über sein C-terminales Ende mit einem Protein von Interesse fusioniert ist, wobei das Verfahren die Trennung des Fusionsproteins Protein N-Protein von Interesse von dem Komplex Protein N-Protein von Interesse /Protein P gemäß einem der Ansprüche 4 bis 8 umfasst.

10. Isoliertes, als lösliche Ringe strukturiertes Fusionsprotein Protein N-Protein von Interesse umfassend das Fusionsprotein Protein N-Protein von Interesse mit seiner RNA, erhältlich durch das Verfahren gemäß Anspruch 9, oder das Protein N ist ein natives oder in dem durch die letzten 25 C-terminalen Aminosäuren definierten Bereich modifiziertes Protein N eines Respiratorischen Syncytial-Viruses und das Protein von Interesse ist an das C-terminale Ende des Proteins N fusioniert.

11. Pharmazeutische Zusammensetzung umfassend ein Protein N eines Respiratorischen Syncytial-Viruses, das mit einem gemäß Anspruch 10 definierten Protein von Interesse oder einem gemäß einem der Ansprüche 4 bis 8 definierten Komplex Protein N-Protein von Interesse /Protein P fusioniert ist, und einen pharmazeutisch annehmbaren Trägerstoff.

12. Die pharmazeutische Zusammensetzung gemäß Anspruch 11, die eine immunogene oder Impfstoff-Zusammensetzung ist.

13. Diagnostisches Reagenz umfassend ein Fusionsprotein gemäß Anspruch 10, wobei das Protein von Interesse ein Antigen ist.

14. Verwendung eines Fusionsproteins gemäß Anspruch 10 umfassend ein an das C-terminale Ende eines Proteins N eines Respiratorischen Syncytial-Viruses fusioniertes Antigen zum *in vitro* Nachweis von Antikörpern, die gegen das Antigen des Fusionsproteins gerichtet sind.

15. Verfahren zum Nachweis von für ein Antigen spezifischen Antikörpern in einer biologischen Probe, umfassend die Schritte bestehend aus:
a) In-Kontakt-bringen der biologischen Probe mit einem Fusionsprotein gemäß Anspruch 10, umfassend ein an das C-terminale Ende eines Proteins N eines Respiratorischen Syncytial-Viruses fusioniertes Antigen,
b) Nachweisen der gebildeten Komplexe Fusionsprotein N-Antigen/Antikörper, wobei die Anwesenheit dieser Komplexe gleichbedeutend mit der Anwesenheit von für das Antigen spezifischen Antikörpern in der Probe ist.

## Claims

1. A method for preparing a N protein-protein of interest/P protein complex, the N and P proteins being proteins of a respiratory syncytial virus (RSV), said method comprising the steps consisting in:
a) coexpressing a protein N of a respiratory syncytial virus - protein of interest fusion protein, with a truncated P protein of the same respiratory syncytial virus;
wherein the protein of interest is fused in frame at the C-terminal end of the N protein, the N protein being a native protein or a N protein modified in the region defined by the last 25 C-terminal amino acids, said N protein retaining the ability to interact with the P protein,
the truncated P protein not containing the oligomerization domain P and comprising a binding domain to the N protein;
b) collecting the so formed N protein-protein of interest/P protein complexes.

2. The method according to claim 1, wherein the respiratory syncytial virus is human respiratory syncytial virus.

3. The method according to claim 1 or claim 2, wherein the truncated P protein is expressed in the form of a fusion protein with a protein that facilitates the purification of the N protein-protein of interest/P protein complexes.

4. N protein-protein of interest/P protein complex in the form of soluble rings comprising the protein N -protein of interest fusion protein with their RNA, the N and P proteins being proteins of a respiratory syncytial virus, wherein the protein of interest is fused in frame at the C-terminal end of the N protein, the N protein being a native protein or a N protein modified in the region defined by the last 25 C-terminal amino acids, said modified N protein retaining the ability to interact with the P protein,
the P protein being a truncated P of the same respiratory syncytial virus not containing the oligomerization domain P and comprising a binding domain to the N protein.

5. N protein-protein of interest/P protein complex according to claim 4, wherein the protein of interest is a marker protein or a protein of vaccinal interest.

6. N protein-protein of interest/P protein complex according to claim 5, wherein the protein of vaccinal interest is an antigen derived from a pathogenic microorganism.

7. N protein-protein of interest/P protein complex according to claim 5, wherein the protein of interest is a construction comprising GFP fused in frame, at its C-terminal end, with a vaccinal protein.

8. N protein-protein of interest/P protein complex according to claim 4, wherein the N protein-protein of interest fusion protein is a N protein-GFP-protein of interest fusion protein comprising GFP (Green Fluorescent Protein) fused in frame at the C-terminal end of N protein, including a linker sequence between the N and GFP proteins having the sequence SEQ ID NO:32.

9. A method for preparing a N protein of a respiratory syncytial virus fused in frame at its C-terminal end with a protein of interest, said method comprising the separation of the N protein-protein of interest fusion protein starting from the N protein-protein of interest/P protein complex according any one of claims 4 to 8.

10. A N protein-protein of interest fusion protein, isolated, in the form of soluble rings comprising the protein N -protein of interest fusion protein with their RNA, obtainable by the method according to claim 9, wherein the N protein is a native protein of a respiratory syncytial virus or is modified in the region defined by the last 25 C-terminal amino acids, and the protein of interest is fused in frame at the C-terminal end of the N protein.

11. A pharmaceutical composition comprising a N protein of a respiratory syncytial virus fused with a protein of interest as defined in claim 10 or N protein-protein of interest/P protein complex as defined in any one of claims 4 to 8, and a pharmaceutically acceptable carrier.

12. The pharmaceutical composition according to claim 11, which is an immunogenic or vaccinal composition.

13. A diagnostic reagent comprising a fusion protein according to claim 10, wherein the protein of interest is an antigen.

14. Use of a fusion protein according to claim 10, comprising an antigen fused in frame at the C-terminal end of a N protein of a respiratory syncytial virus in the *in vitro* detection of antibodies directed against said antigen of the fusion protein.

15. A method for detecting antibodies specific for an antigen in a biological sample, which method comprises the steps consisting in:
a) contacting said biological sample with a fusion protein according to claim 10, comprising an antigen fused in frame at the C-terminal end of a N protein of a respiratory syncytial virus,
b) detecting the resulting N-antigen fusion protein/antibody complexes,
the presence of such complexes being indicative of the presence in the biological sample of antibodies specific for the antigen.
